# EUROPEAN PATENT SPECIFICATION

(11) **EP 1 012 161 B1**
(45) Date of publication and mention of the grant of the patent: **16.03.2011**
(21) Application number: 98943434.5
(22) Date of filing: 28.08.1998
(51) Int. Cl.: C07H 21/04, C12N 9/12, C12P 19/34

(54) **HIGH FIDELITY POLYMERASES AND USES THEREOF**
HOCHEMPFINDLICHE POLYMERASEN UND IHRE VERWENDUNGEN
POLYMERASES HAUTE FIDELITE ET LEUR UTILISATION

(30) Priority: 29.08.1997 US 56263 P; 26.09.1997 US 60131 P; 13.05.1998 US 85247 P; 27.08.1998 US 141522
(43) Date of publication of application: 28.06.2000
(60) Divisional application: 10178608.5
(73) Proprietor: Life Technologies Corporation, Carlsbad, CA 92008 (US)
(72) Inventor: YANG, Shuwei, Rockville, MD 20855 (US); CHATTERJEE, Deb, K., North Potomac, MD 20878 (US)
(74) Representative: Williams, Richard Andrew Norman
(86) International application number: PCT/US1998/017810
(87) International publication number: WO 1999/010366

(56) References cited:
- WO-A-96/10640
- WO-A-97/09451
- WO-A-98/23733
- WO-A1-95/33853
- WO-A1-96/41014
- ASTATKE M ET AL: "Deoxynucleoside triphosphate and pyrophosphate binding site in the catalytically competent ternary complex for the polymerase reaction catalyzed by DNA polymerase I (Kleno fragment)" JOURNAL OF BIOLOGICAL CHEMISTRY, AMERICAN SOCIETY OF BIOLOGICAL CHEMISTS, BALTIMORE, MD, US, vol. 270, no. 4, 27 January 1995 (1995-01-27), pages 1945-1954, XP002253151 ISSN: 0021-9258
- SUZUKI M ET AL: "RANDOM MUTAGENESIS OF THERMUS AQUATICUS DNA POLYMERASE I: CONCORDANCE OF IMMUTABLE SITES IN VIVO WITH THE CRYSTAL STRUCTURE" PROCEEDINGS OF THE NATIONAL ACADEMY OF SCIENCES OF USA, NATIONAL ACADEMY OF SCIENCE. WASHINGTON, US, vol. 93, 1 September 1996 (1996-09-01), pages 9670-9675, XP002062865 ISSN: 0027-8424
- NEWCOMB T G ET AL: "HIGH FIDELITY TAQ POLYMERASES FOR MUTATION DETECTION" FASEB JOURNAL, FED. OF AMERICAN SOC. FOR EXPERIMENTAL BIOLOGY, BETHESDA, MD, US, vol. 9, no. 11, 24 August 1997 (1997-08-24), page A1249, XP002062867 ISSN: 0892-6638
- PANDEY et al., "Role of Methionine 184 of Human Immunodeficiency Virus Type-1 Reverse Transcriptase in the Polymerase Function and Fidelity of DNA Synthesis", BIOCHEMISTRY, February 1996, Vol. 35, No. 7, pages 2168-2179, XP002915505
- DONG et al., "Mutational Studies of Human DNA Polymerase alpha", J. BIOL. CHEM., 15 September 1995, Vol. 270, No. 37, pages 21563-21570, XP002915506
- BARNES W.M., "The Fidelity of Taq Polymerase Catalyzing PCR is Improved by an N-Terminal Deletion", GENE, April 1992, Vol. 112, pages 29-35, XP002915507
- EGER et al., "Mechanism of DNA Replication Fidelity for Three Mutants of DNA Polymerase I: Klenow Fragment KF(exo+), KF(polA5) and KF(exo)", BIOCHEMISTRY, 05 February 1991, Vol. 30, No. 5, pages 1441-1448, XP002915508
- BEBENEK et al., "The Fidelity of DNA Synthesis Catalyzed by Derivatives of Escherichia Coli DNA Polymerase I", J. BIOL. CHEM., August 1990, Vol. 265, No. 23, pages 13878-13887, XP002915509
- DROSOPOULOS et al., "Increased Polymerase Fidelity of E89G, a Nucleoside Analog-Resistant Variant of Human Immunodeficiency Virus Type 1 Reverse Transcriptase", J. VIROLOGY, July 1996, Vol. 70, No. 7, pages 4834-4838, XP002915510

## Description

### BACKGROUND OF THE INVENTION

### Field of the Invention

The present invention relates to substantially pure Tne DNA polymerases having high fidelity. Specifically, the polymerases of the present invention are Tne DNA polymerases which have been mutated or modified to increase the fidelity of the polymerase (compared to the unmodified or unmutated polymerase), thereby providing a polymerase which has a lower misincorporation rate (reduced misincorporation). Preferably, the polymerases are thermostable or mesophilic polymerases. The present invention also relates to cloning and expression of the polymerases of the invention, to DNA molecules containing the cloned gene, and to hosts which express said genes. The polymerases of the present invention may be used in DNA sequencing, amplification reactions, nucleic acid synthesis and cDNA synthesis. The invention also relates to polymerases of the invention which have one or more additional mutations or modifications. Such mutations or modifications include those which (1) substantially reduce 3'→5' exonuclease activity; (2) enhance or increase the ability of the polymerase to incorporate dideoxynucleotides into a DNA molecule about as efficiently as deoxynucleotides; and (3) substantially reduce 5'→3' exonuclease activity. The polymerases of this invention can have one or more of these properties. These polymerases may also be used in DNA sequencing, amplification reactions, nucleic acid synthesis and cDNA synthesis.

### Related Art

DNA polymerases synthesize the formation of DNA molecules which are complementary to a DNA template. Upon hybridization of a primer to the single-stranded DNA template, polymerases synthesize DNA in the 5' to 3' direction, successively adding nucleotides to the 3'-hydroxyl group of the growing strand. Thus, in the presence of deoxyribonucleoside triphosphates (dNTPs) and a primer, a new DNA molecule, complementary to the single stranded DNA template, can be synthesized.

A number of DNA polymerases have been isolated from mesophilic microorganisms such as *E. coli.* A number of these mesophilic DNA polymerases have also been cloned. Lin *et al.* cloned and expressed T4 DNA polymerase in *E. coli* (Proc. Natl. Acad. Sci. USA 84:7000-7004 (1987)). Tabor et al. (U.S. Patent No. 4,795,699) describes a cloned T7 DNA polymerase, while Minkley et al. (J. Biol. Chem. 259:10386-10392 (1984)) and Chatterjee (U.S. Patent No. 5,047,342) described *E. coli* DNA polymerase I and the cloning of T5 DNA polymerase, respectively.

DNA polymerases from thermophiles have also been described. Chien et al., J. Bacteriol. 127:1550-1557 (1976) describe a purification scheme for obtaining a polymerase from *Thermus aquaticus (Taq).* The resulting protein had a molecular weight of about 63,000 daltons by gel filtration analysis and 68,000 daltons by sucrose gradient centrifugation. Kaledin et al., Biokhymiya 45:644-51 (1980) disclosed a purification procedure for isolating DNA polymerase from *T. aquaticus* YT1 strain. The purified enzyme was reported to be a 62,000 dalton monomeric protein. Gelfand et al. (U.S. Patent No. 4,889,818) cloned a gene encoding a thermostable DNA polymerase from *Thermus aquaticus.* The molecular weight of this protein was found to be about 86,000 to 90,000 daltons. Simpson *et al.* purified and partially characterized a thermostable DNA polymerase from a *Thermotoga* species (Biochem. Cell. Biol. 86:1292-1296 (1990)). The purified DNA polymerase isolated by Simpson *et al.* exhibited a molecular weight of 85,000 daltons as determined by SDS-polyacrylamide gel electrophoresis and size-exclusion chromatography. The enzyme exhibited half-lives of 3 minutes at 95°C and 60 minutes at 50°C in the absence of substrate and its pH optimum was in the range of pH 7.5 to 8.0. Triton X-100 appeared to enhance the thermostability of this enzyme. The strain used to obtain the thermostable DNA polymerase described by Simpson *et al.* was *Thermotoga* species strain FjSS3-B.1 (Hussar et al., FEMS Microbiology Letters 37:121-127 (1986)). Others have cloned and sequenced a thermostable DNA polymerase from *Thermotoga maritima* (U.S. Patent 5,374,553).

Other DNA polymerases have been isolated from thermophilic bacteria including *Bacillus sterothermophilus* (Stenesh et al., Biochim. Biophys. Acta 272:156-166 (1972); and Kaboev et al., J. Bacteriol. 145:21-26 (1981)) and several archaebacterial species (Rossi et al., System. Appl. Microbiol. 7:337-341 (1986); Klimczak et al., Biochemistry 25:4850-4855 (1986); and Elie et al., Eur. J. Biochem. 178:619-626 (1989)). The most extensively purified archaebacterial DNA polymerase had a reported half-life of 15 minutes at 87°C (Elie *et al.* (1989), supra). Innis et al., In PCR Protocol: A Guide To Methods and Amplification, Academic Press, Inc., San Diego (1990) noted that there are several extreme thermophilic eubacteria and archaebacteria that are capable ofgrowth at very high temperatures (Bergquist et al., Biotech. Genet. Eng. Rev. 5:199-244 (1987); and Kelly et al., Biotechnol. Prog. 4:47-62 (1988)) and suggested that these organisms may contain very thermostable DNA polymerases.

In many of the known polymerases, the 5'→3' exonuclease activity is present in the N-terminal region of the polymerase. (Ollis, et al., Nature 313:762-766 (1985); Freemont et al., Proteins 1:66-73 (1986); Joyce, Cur. Opin. Struct. Biol. 1:123-129 (1991).) There are some amino acids, the mutation of which are thought to impair the 5'→3' exonuclease activity of *E*. *coli* DNA polymerase I. (Gutman & Minton, Nucl. Acids Res. 21:4406-4407 (1993).) These amino acids include Tyr⁷⁷, Gly¹⁰³, Gly¹⁸⁴, and Gly¹⁹² in *E. coli* DNA polymerase I. It is known that the 5'-exonuclease domain is dispensable. The best known example is the Klenow fragment of E. *coli* polymerase I. The Klenow fragment is a natural proteolytic fragment devoid of 5'-exonuclease activity (Joyce et. al., J. Biol. Chem. 257:1958-64 (1990).) Polymerases lacking this activity are useful for DNA sequencing.

Most DNA polymerases also contain a 3'→ 5' exonuclease activity. This exonuclease activity provides a proofreading ability to the DNA polymerase. A T5 DNA polymerase that lacks 3' → 5' exonuclease activity is disclosed in U.S. Patent No. 5,270,179. Polymerases lacking this activity are particularly useful for DNA sequencing.

The polymerase active site, including the dNTP binding domain is usually present at the carboxyl terminal region of the polymerase (Ollis et al., Nature 313:762-766 (1985); Freemont et al., Proteins 1:66-73 (1986)). It has been shown that Phe⁷⁶² of *E. Coli* polymerase I is one of the amino acids that directly interacts with the nucleotides (Joyce & Steitz, Ann. Rev. Biochem. 63:777-822 (1994); Astatke, J Biol. Chem. 270:1945-54(1995)). Converting this amino acid to a Tyr results in a mutant DNA polymerase that does not discriminate against dideoxynucleotides. See U.S. Patent 5,614,365.

WO 97/09451 describes cloning of DNA polymerases from *Themotogo* (*Tne* and *Tma*) and mutants or combinations of mutants. One mutation may substantially reduce or eliminate the 3'→5' exonuclease activity of the polymerase. Another mutation may substantially reduce or eliminate 5'→ 3' exonuclease activity of the polymerase. A further mutation in the O helix results in the polymerase becoming non-discriminating against dideoxynucleotides.

Finally, Suzuki et al PNAS, VOL. 93, 1996, pp. 9670-9675 and Newcomb et al FASEB J, vol. 9, 1997, pA1249, disclose the random mutagenesis of *Thermus aquaticus (Taq)* DNA polymerase. Suzuki *et al* teach that residues Arg659 and Lys663 of the O-helix of the polymerase are immutable.

While polymerases are known, there exists a need in the art to develop polymerases which are more suitable for nucleic acid synthesis, sequencing, and amplification. Such polymerases would have reduced error rate; that is reduced misincorporation of nucleotides during nucleic acid synthesis and/or increased fidelity of polymerization.

### BRIEF SUMMARY OF THE INVENTION

The present invention satisfies these needs in the art by providing additional polymerases useful in molecular biology. The invention therefore provides a *Tne* DNA polymerase as set forth in claims 1 to 12. These polymerases are modified or mutated in their nucleotide binding domain within the enzyme such that the fidelity of the enzyme is increased or enhanced. The nucleotide binding domain of a polymerase is typically referred to as the O-helix.

The present invention also provides vectors as set forth in claim 15 on claim 16, and host cells as set forth in claim 17. Any number of hosts may be used to express the gene of interest, including prokaryotic and eukaryotic cells. Preferably, prokaryotic cells are used to express the polymerases of the invention. The preferred prokaryotic host according to the present invention is *E*. *coli.*

The invention also provides a method of producing the polymerases of the invention, said method comprising:
(a) culturing the host cell comprising a gene encoding the polymerases of the invention;
(b) expressing said gene; and
(c) isolating said polymerase from said host cell.

The invention also provides a method of synthesizing a nucleic acid molecule comprising:
(a) mixing a nucleic acid template (e.g. RNA or DNA) with one or more polymerases of the invention; and
(b) incubating said mixture under conditions sufficient to synthesize a nucleic acid molecule complementary to all or a portion of said template. Such condition may include incubation with one or more deoxy- or dideoxyribonucleoside triphosphates. Such deoxy- and dideoxyribonucleoside triphosphates include dATP, dCTP, dGTP, dTTP, dITP, 7-deaza-dGTP, 7-deaza-dATP, dUTP, ddATP, ddCTP, ddGTP, ddITP, ddTTP, [α-S]dATP, [α-S]dTTP, [α-S]dGTP, and [α-S]dCTP.

The invention also provides to a method of sequencing a DNA molecule, comprising:
(a) hybridizing a primer to a first DNA molecule;
(b) contacting said molecule of step (a) with deoxyribonucleoside triphosphates, a DNA polymerase of the invention, and a terminator nucleotide;
(c) incubating the mixture of step (b) under conditions sufficient to synthesize a random population of DNA molecules complementary to said first DNA molecule, wherein said synthesized DNA molecules are shorter in length than said first DNA molecule and wherein said synthesized DNA molecules comprise a terminator nucleotide at their 3' termini; and
(d) separating said synthesized DNA molecules by size so that at least a part of the nucleotide sequence of said first DNA molecule can be determined. Such terminator nucleotides may be include ddTTP, ddATP, ddGTP, ddITP or ddCTP.

The invention also provides to a method for amplifying a double stranded DNA molecule, comprising:
(a) providing a first and second primer, wherein said first primer is complementary to a sequence at or near the 3'-termini of the first strand of said DNA molecule and said second primer is complementary to a sequence at or near the 3'-termini of the second strand of said DNA molecule;
(b) hybridizing said first primer to said first strand and said second primer to said second strand in the presence of the DNA polymerase of the invention, under conditions such that a third DNA molecule complementary to said first strand and a fourth DNA molecule complementary to said second strand are synthesized;
(c) denaturing said first and third strand, and said second and fourth strands; and
(d) repeating steps (a) to (c) one or more times.

The invention also provides a kit for sequencing, amplifying or synthesis of a nucleic acid molecule comprising one or more polymerases of the invention. The kit may include one or more other components selected from the group consisting of
(a) one or more dideoxyribonucleoside triphosphates;
(b) one or more deoxyribonucleoside triphosphates;
(c) one or more primers; and
(d) one or more suitable buffers.

Other preferred embodiments of the present invention will be apparent to one of ordinary skill in light of the following drawings and description of the invention, and of the claims.

### BRIEF DESCRIPTION OF THE DRAWINGS

**FIG.1** shows the restriction map of the approximate DNA fragment which contains the *Tne* DNA polymerase gene in pSport 1 and pUC19. This figure also shows the region containing the O-helix homologous sequences.
**FIG. 2A** schematically depicts the construction of plasmids pUC-Tne (3'→5') and pUC-Tne FY.
**FIG. 2B** schematically depicts the construction of plasmids pTrc Tne35 and pTrcTne FY.
**FIG. 3** schematically depicts the construction of plasmid pTrcTne35 FY.
**FIG. 4** schematically depicts the construction of plasmid pTTQTne5FY and pTTQTne535FY.

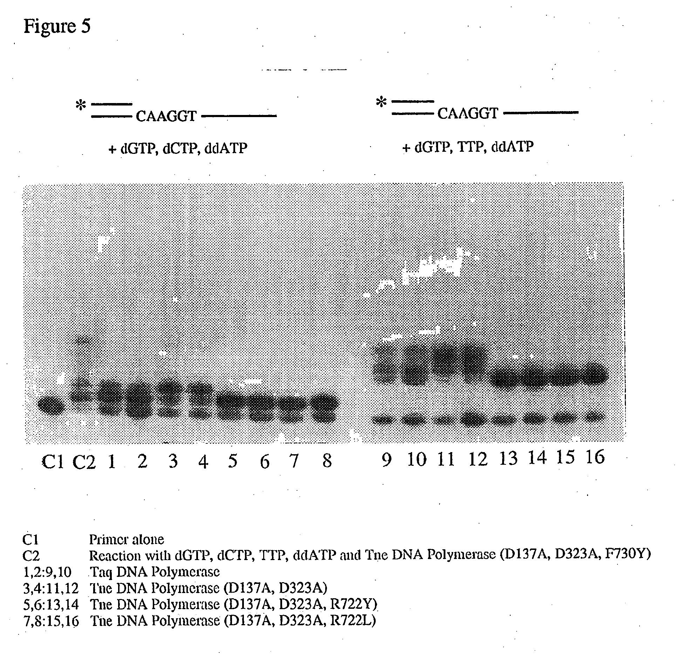

**FIG. 5** depicts the primer extension assay showing increase in fidelity and decrease in misincorporation of mutant polymerases of the invention.

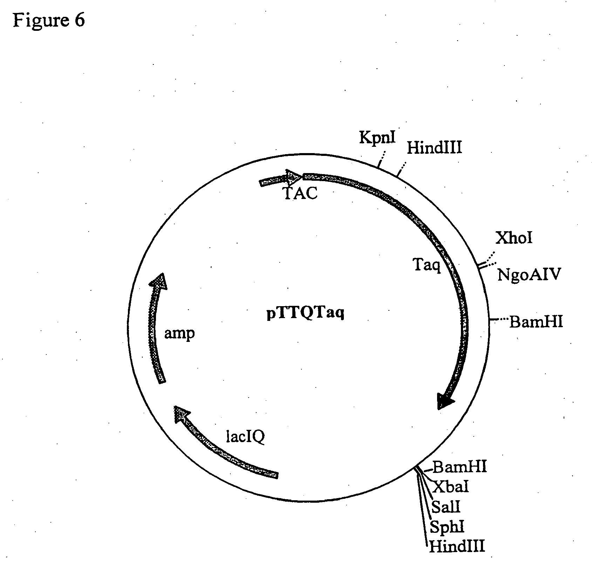

**FIG. 6** depicts a plasmid containing the Taq DNA polymerase gene.

### DETAILED DESCRIPTION OF THE INVENTION

### Definitions

In the description that follows, a number of terms used in recombinant DNA technology are extensively utilized. In order to provide a clearer and consistent understanding of the specification and claims, including the scope to be given such terms, the following definitions are provided.

**Cloning vector.** A plasmid, cosmid or phage DNA or other DNA molecule which is able to replicate autonomously in a host cell, and which is characterized by one or a small number of restriction endonuclease recognition sites at which such DNA sequences may be cut in a determinable fashion without loss of an essential biological function of the vector, and into which DNA may be spliced in order to bring about its replication and cloning. The cloning vector may further contain a marker suitable for use in the identification of cells transformed with the cloning vector. Markers, for example, are tetracycline resistance or ampicillin resistance.

**Expression vector.** A vector similar to a cloning vector but which is capable of enhancing the expression of a gene which has been cloned into it, after transformation into a host. The cloned gene is usually placed under the control of (i.e., operably linked to) certain control sequences such as promoter sequences.

**Recombinant host.** Any prokaryotic or eukaryotic or microorganism which contains the desired cloned genes in an expression vector, cloning vector or any DNA molecule. The term "recombinant host" is also meant to include those host cells which have been genetically engineered to contain the desired gene on the host chromosome or genome.

**Host.** Any prokaryotic or eukaryotic microorganism that is the recipient of a replicable expression vector, cloning vector or any DNA molecule. The DNA molecule may contain, but is not limited to, a structural gene, a promoter and/or an origin of replication.

**Promoter.** A DNA sequence generally described as the 5' region of a gene, located proximal to the start codon. At the promoter region, transcription of an adjacent gene(s) is initiated.

**Gene.** A DNA sequence that contains information necessary for expression of a polypeptide or protein. It includes the promoter and the structural gene as well as other sequences involved in expression of the protein.

**Structural gene.** A DNA sequence that is transcribed into messenger RNA that is then translated into a sequence of amino acids characteristic of a specific polypeptide.

Operably linked. As used herein means that the promoter is positioned to control the initiation of expression of the polypeptide encoded by the structural gene.

**Expression.** Expression is the process by which a gene produces a polypeptide. It includes transcription of the gene into messenger RNA (mRNA) and the translation of such mRNA into polypeptide(s).

**Substantially Pure.** As used herein "substantially pure" means that the desired purified protein is essentially free from contaminating cellular contaminants which are associated with the desired protein in nature. Contaminating cellular components may include, but are not limited to, phosphatases, exonucleases, endonucleases or undesirable DNA polymerase enzymes.

**Primer.** As used herein "primer" refers to a single-stranded oligonucleotide that is extended by covalent bonding of nucleotide monomers during amplification or polymerization of a DNA molecule.

**Template.** The term "template" as used herein refers to a double-stranded or single-stranded DNA molecule which is to be amplified, synthesized or sequenced. In the case of a double-stranded DNA molecule, denaturation of its strands to form a first and a second strand is performed before these molecules may be amplified, synthesized or sequenced. A primer, complementary to a portion of a DNA template is hybridized under appropriate conditions and the DNA polymerase of the invention may then synthesize a DNA molecule complementary to said template or a portion thereof. The newly synthesized DNA molecule, according to the invention, may be equal or shorter in length than the original DNA template. Mismatch incorporation during the synthesis or extension of the newly synthesized DNA molecule may result in one or a number of mismatched base pairs. Thus, the synthesized DNA molecule need not be exactly complementary to the DNA template.

**Incorporating.** The term "incorporating" as used herein means becoming a part of a DNA molecule or primer.

**Amplification.** As used herein "amplification" refers to any *in vitro* method for increasing the number of copies of a nucleotide sequence with the use of a DNA polymerase. Nucleic acid amplification results in the incorporation of nucleotides into a DNA molecule or primer thereby forming a new DNA molecule complementary to a DNA template. The formed DNA molecule and its template can be used as templates to synthesize additional DNA molecules. As used herein, one amplification reaction may consist of many rounds of DNA replication. DNA amplification reactions include, for example, polymerase chain reactions (PCR). One PCR reaction may consist of 20 to 100 "cycles" of denaturation and synthesis of a DNA molecule.

**Oligonucleotide.** "Oligonucleotide" refers to a synthetic or natural molecule comprising a covalently linked sequence of nucleotides which are joined by a phosphodiester bond between the 3' position of the pentose of one nucleotide and the 5' position of the pentose of the adjacent nucleotide.

**Nucleotide.** As used herein "nucleotide" refers to a base-sugar-phosphate combination. Nucleotides are monomeric units of a nucleic acid sequence (DNA and RNA). The term nucleotide includes deoxyribonucleoside triphosphates such as dATP, dCTP, dITP, dUTP, dGTP, dTTP, or derivatives thereof. Such derivatives include, for example, [αS]dATP, 7-deaza-dGTP and 7-deaza-dATP. The term nucleotide as used herein also refers to dideoxyribonucleoside triphosphates (ddNTPs) and their derivatives. Illustrated examples of dideoxyribonucleoside triphosphates include, but are not limited to, ddATP, ddCTP, ddGTP, ddITP, and ddTTP. According to the present invention, a "nucleotide" may be unlabeled or detectably labeled by well known techniques. Detectable labels include, for example, radioactive isotopes, fluorescent labels, chemiluminescent labels, bioluminescent labels and enzyme labels.

**Thermostable.** As used herein "thermostable" refers to a DNA polymerase which is resistant to inactivation by heat. DNA polymerases synthesize the formation of a DNA molecule complementary to a single-stranded DNA template by extending a primer in the 5'-to-3' direction. This activity for mesophilic DNA polymerases may be inactivated by heat treatment. For example, T5 DNA polymerase activity is totally inactivated by exposing the enzyme to a temperature of 90°C for 30 seconds. As used herein, a thermostable DNA polymerase activity is more resistant to heat inactivation than a mesophilic DNA polymerase. However, a thermostable DNA polymerase does not mean to refer to an enzyme which is totally resistant to heat inactivation and thus heat treatment may reduce the DNA polymerase activity to some extent. A thermostable DNA polymerase typically will also have a higher optimum temperature than mesophilic DNA polymerases.

**Hybridization.** The terms "hybridization" and "hybridizing" refers to the pairing oftwo complementary single-stranded nucleic acid molecules (RNA and/or DNA) to give a double-stranded molecule. As used herein, two nucleic acid molecules may be hybridized, although the base pairing is not completely complementary. Accordingly, mismatched bases do not prevent hybridization of two nucleic acid molecules provided that appropriate conditions, well known in the art, are used.

**3'-to-5' Exonuclease Activity.** "3'-to-5' exonuclease activity" is an enzymatic activity well known to the art. This activity is often associated with DNA polymerases, and is thought to be involved in a DNA replication "editing" or correction mechanism.

A "DNA polymerase substantially reduced in 3'-to-5' exonuclease activity" is defined herein as either (1) a mutated DNA polymerase that has about or less than 10%, or preferably about or less than 1%, of the 3'-to-5' exonuclease activity of the corresponding unmutated, wild-type enzyme, or (2) a DNA polymerase having a 3'-to-5' exonuclease specific activity which is less than about 1 unit/mg protein, or preferably about or less than 0.1 units/mg protein. A unit of activity of 3'-to-5' exonuclease is defined as the amount of activity that solubilizes 10 nmoles of substrate ends in 60 min. at 37°C, assayed as described in the "BRL 1989 Catalogue & Reference Guide", page 5, with *Hha*I fragments of *lambda* DNA 3'-end labeled with [³H]dTTP by terminal deoxynucleotidyl transferase (TdT). Protein is measured by the method of Bradford, Anal. Biochem. 72:248 (1976). As a means of comparison, natural, wild-type T5-DNA polymerase (DNAP) or T5-DNAP encoded by pTTQ19-T5-2 has a specific activity of about 10 units/mg protein while the DNA polymerase encoded by pTTQ19-T5-2(Exo⁻) (U.S. 5,270,179) has a specific activity of about 0.0001 units/mg protein, or 0.001% of the specific activity of the unmodified enzyme, a 10⁵-fold reduction.

**5'-to-3' Exonuclease Activity.** "5'-to-3' exonuclease activity" is also an enzymatic activity well known in the art. This activity is often associated with DNA polymerases, such as *E. coli* PolI and PolIII.

A "DNA polymerase substantially reduced in 5'-to-3' exonuclease activity" is defined herein as either (1) a mutated DNA polymerase that has about or less than 10%, or preferably about or less than 1%, of the 5'-to-3' exonuclease activity of the corresponding unmutated, wild-type enzyme, or (2) a DNA polymerase having 5'-to-3' exonuclease specific activity which is less than about 1 unit mg protein, or preferably about or less than 0.1 units/mg protein.

Both of the 3'-to-5' and 5'-to-3' exonuclease activities can be observed on sequencing gels. Active 5'-to-3' exonuclease activity will produce nonspecific ladders in a sequencing gel by removing nucleotides from the 5'-end of the growing primers. 3'-to-5' exonuclease activity can be measured by following the degradation of radiolabeled primers in a sequencing gel. Thus, the relative amounts of these activities, e.g. by comparing wild-type and mutant polymerases, can be determined with no more than routine experimentation.

**Fidelity.** Fidelity refers to the accuracy of polymerization, or the ability of the polymerase to discriminate correct from incorrect substrates, (e.g., nucleotides) when synthesizing nucleic acid molecules (e.g. RNA or DNA) which are complementary to a template. The higher the fidelity of a polymerase, the less the polymerase misincorporates nucleotides in the growing strand during nucleic acid synthesis; that is, an increase or enhancement in fidelity results in a more faithful polymerase having decreased error rate (decreased misincorporation rate).

A DNA polymerase having increased/enhanced/higher fidelity is defined as a polymerase having about 2 to about 10,000 fold, about 2 to about 5,000 fold, or about 2 to about 2000 fold (preferably greater than about 5 fold, more preferably greater than about 10 fold, still more preferably greater than about 50 fold, still more preferably greater than about 100 fold, still more preferably greater than about 500 fold and most preferably greater than about 100 fold) reduction in the number of misincorporated nucleotides during synthesis of any given nucleic acid molecule of a given length. For example, a mutated polymerase may misincorporate one nucleotide in the synthesis of 1000 bases compared to an unmutated polymerase miscincorporating 10 nucleotides. Such a mutant polymerase would be said to have an increase of fidelity of 10 fold.

A DNA polymerase having reduced misincorporation is defined herein as either a mutated or modified DNA polymerase that has about or less than 50%, or preferably about or less than 25%, more preferably about or less than 10% and most preferably about or less than 1% of relative misincorporation compared to the corresponding unmutated, unmodified or wild type enzyme. A less fidelity DNA polymerase may also initiate DNA synthesis with an incorrect nucleotide incorporation (Perrion & Loeb, 1989, J. Biol. Chem. 264:2898-2905).

The fidelity or misincorporation rate of a polymerase can be determined by sequencing or by other method known in the art (Eckert & Kunkel, 1990, Nuc. Acids Res., 3739-3744). In one example, the sequence of a DNA molecule synthesized by the unmutated and mutated polymerase can be compared to the expected (known) sequence. In this way, the number of errors (misincorporation) can be determined for each enzyme and compared. In another example, the unmutated and mutated polymerase may be used to sequence a DNA molecule having a known sequence. The number of sequencing errors (misincorporation) can be compared to determine the fidelity or misincorporation rate of the enzymes. Other means of determining the fidelity or misincorporation rate will be recognized by one of skill in the art.

### Sources of Polymerases

*Thermotoga neapolitana (Tne)* DNA polymerase and mutants, and variants and derivatives thereof are used in accordance with the invention.

For amplification of long nucleic acid molecules (e.g., nucleic acid molecules longer than about 3-5 Kb in length), at least two DNA polymerases (one substantially lacking 3' exonuclease activity and the other having 3' exonuclease activity) are typically used. *See* U.S. Patent No. 5,436,149; U.S. Patent No. 5,512,462; Barnes, W.M., Gene 112:29-35 (1992); and copendingU.S. Patent Application No. 08/689,814, filed February 14, 1997. Examples of DNA polymerases substantially lacking in 3' exonuclease activity include, but are not limited to, *Taq, Tne*(exo), *Tma*(exo⁻), *Pfu* (exo⁻), *Pwo*(exo⁻) and *Tth* DNA polymerases, and mutants, variants and derivatives thereof.

The modified or mutant polymerase of the invention is preferably used in the present methods at a final concentration in solution of about 0.1-200 units per milliliter, about 0.1-50 units per milliliter, about 0.1-40 units per milliliter, about 0.1-3.6 units per milliliter, about 0.1-34 units per milliliter, about 0.1-32 units per milliliter, about 0.1-30 units per milliliter, or about 0.1-20 units per milliliter, and most preferably at a concentration of about 20 units per milliliter. Of course, other suitable concentrations of the nucleic acid polymerase suitable for use in the invention will be apparent to one or ordinary skill in the art.

In a preferred aspect of the invention, the mutant or modified polymerase is made by recombinant techniques. A number of cloned polymerase genes are available or may be obtained using standard recombinant techniques.

To clone a gene encoding a DNA polymerase which will be modified in accordance with the invention, isolated DNA which contains the polymerase gene is used to construct a recombinant DNA library in a vector. Any vector, well known in the art, can be used to clone the DNA polymerase of interest. However, the vector used must be compatible with the host in which the recombinant DNA library will be transformed.

Prokaryotic vectors for constructing the plasmid library include plasmids such as those capable of replication in *E. coli* such as, for example, pBR322, ColE1, pSC101, pUC-vectors (pUC18, pUC19, etc.: In: Molecular Cloning, A Labaratory Manual, Cold Spring Harbor Laboratory Press, Cold Spring Harbor, New York (1982); and Sambrook et al., In: Molecular Cloning A Laboratory Manual (2d ed.) Cold Spring Harbor Laboratory Press, Cold Spring Harbor, New York (1989)). Bacillus plasmids include pC194, pC221, pC217, etc. Such plasmids are disclosed by Glyczan, T. In: The Molecular Biology Bacilli, Academic Press, York (1982), 307-329. Suitable *Streptomyces* plasmids include pIJ101 (Kendall et al., J. Bacteriol 169:4177-4183 (1987)). *Pseudomonas* plasmids are reviewed by John et al., (Rad. Insec. Dis. 8:693-704 (1986)), and Igaki, (Jpn. J. Bacteriol. 33:729-742 (1978)). Broad-host range plasmids or cosmids, such as pCP13 (Darzins and Chakrabarbarty, J. Bacteriol. 159:9-18, 1984) can also be used for the present invention. The preferred vectors for cloning the genes of the present invention are prokaryotic vectors. Preferably, pCP13 and pUC vectors are used to clone the genes of the present invention.

The preferred host for cloning the polymerase genes of interest is a prokaryotic host. The most preferred prokaryotic host is *E*. *coli.* However, the desired polymerase genes of the present invention may be cloned in other prokaryotic hosts including, but not limited to, *Escherichia, Bacillus, Streptomyces, Pseudomonas, Salmonella, Serratia,* and *Proteus.* Bacterial hosts of particular interest include *E*. *coli* DH10B, which may be obtained from Life Technologies, Inc. (LTI) (Rockville, MD).

Eukaryotic hosts for cloning and expression of the polymerases of interest include yeast, fungi, and mammalian cells. Expression of the desired polymerase in such eukaryotic cells may require the use of eukaryotic regulatory regions which include eukaryotic promoters. Cloning and expressing the polymerase gene in eukaryotic cells may be accomplished by well known techniques using well known eukaryotic vector systems.

Once a DNA library has been constructed in a particular vector, an appropriate host is transformed by well known techniques. Transformed colonies are plated at a density of approximately 200-300 colonies per petri dish. For thermostable polymerase selection, colonies are then screened for the expression of a heat stable DNA polymerase by transferring transformed *E*. *coli* colonies to nitrocellulose membranes. After the transferred cells are grown on nitrocellulose (approximately 12 hours), the cells are lysed by standard techniques, and the membranes are then treated at 95°C for 5 minutes to inactivate the endogenous *E. coli* enzyme. Other temperatures may be used to inactivate the host polymerases depending on the host used and the temperature stability of the polymerase to be cloned. Stable polymerase activity is then detected by assaying for the presence of polymerase activity using well known techniques. Sagner et al., Gene 97:119-123 (1991). The gene encoding a polymerase of the present invention can be cloned using the procedure described by Sagner *et al., supra.*

### Modifications or Mutations of Polymerases

In accordance with the invention, the nucleotide binding domain of the polymerase of interest is modified or mutated in such a way as to produce a mutated or modified polymerase having increased or enhanced fidelity (decreased misincorporation rate). The O-helix region typically defines the nucleotide binding domain of DNA polymerases. The O-helix may be defined as RXXXKXXXFXXXYX (SEQ ID NO:1), wherein X is an amino acid and one modification or mutation is as set forth in claim 1. One or more further mutations may be made in the O-helix of the Tne polymerase in order to increase fidelity of the enzyme in accordance with the invention. Such mutations include point mutations, flame shift mutations, deletions and insertions. Preferably, one or more point mutations, resulting in one or more amino acid substitutions, are used to produce polymerases having enhanced or increased fidelity. In preferred aspects of the invention, one or more mutations at position R, K, F, and/or Y may be made to produced the desired result.

In another particularly preferred aspect, mutations at position R and at position K within the O-helix results in polymerases having increased fidelity and/or reduced misincorporation rate. In this preferred aspect, amino acid substitutions are made at positions R and K. Thus, according to this aspect of the invention K (Lys) may be further substituted with any other amino acid including Ala, Arg, Asn, Asp, Cys, Gln, Glu, Gly, His, Ile, Leu, Met, Phe, Pro, Ser, Thr, Trp, Tyr, and Val.

The O-helix has been identified and defined for a number of polymerases and may be readily identified for other polymerases by one with skill in the art. Thus, given the defined O-helix region and the assays described in the present application, one with skill in the art can make one or a number of modifications which would result in increased fidelity of a polymerase. The following table illustrates identified O-helix regions for known polymerases, where only Tne is of the invention.

| **Polymerase** | **O-helix Region** |
|---|---|
| PolI | 754 RRSAKAINFGLIYG (SEQ ID NO:2) |
| Taq | 659 RRAAKTINFGVLYG (SEQ ID NO:3) |
| T7 | 518 RDNAKTFIYGFLYG (SEQ ID NO:4). |
| Tne | 722 RRVGKMVNFSIIYG (SEQ ID NO: 5) |
| T5 | 588 RQAAKAITFGILYG (SEQ ID NO:6) |
| Tma | 722 RRAGKMVNFSIIYG (SEQ ID NO:7) |

Thus, in accordance with one preferred aspect of the invention; corresponding mutations in the R position and/or the K position of the O-helix can be made for the following enzymes based on the table below, where only Tne is of the invention.

| **Polymerase** | **Mutation Position(s)** |
|---|---|
| PolI | Arg⁷⁵⁴ and/or Lys⁷⁵⁸ |
| T5 | Arg⁵⁹⁸ and/or Lys⁵⁹² |
| T7 | Arg⁵¹⁸ and/or Lys⁵²² |
| Taq | Arg⁶⁵⁹ and/or Lys⁶⁶³ |
| Tne | Arg⁷²² and/or Lys⁷²⁶ |
| Tma | Arg and/or Lys⁷²⁶ |
| Bca | Arg⁷⁰⁵ and/or Lys⁷⁰⁷ |
| Bst | Arg⁷⁰² and/or Lys⁷⁰⁶ |
| Tth | Arg⁶⁶¹ and/or Lys⁶⁶⁵ |

The mutation position of Arg⁷⁰⁵ for *Bca* is based on the sequence information in GenBank. It should be noted, however, that according to the sequence described by Vemori et al., J Biochem. (Japan) 113:401-410 (1993), the position of Arg in *Bca* is 703.

### Additional Modifications or Mutations of Polymerases

In accordance with the invention, in addition to the mutations described above for creating Tne polymerases with lower misincorporation or for enhancing fidelity, one or more additional mutations or modifications (or combinations thereof) may be made to the polymerases of interest. Mutations or modifications of particular interest include those modifications of mutations which (1) reduce or eliminate 3' to 5' exonuclease activity; (2) eliminate or reduce 5' to 3' exonuclease activity; and (3) reduce discrimination of dideoxynucleotides (that is, increase incorporation of dideoxynucleotides).

3'to 5' exonuclease activity be reduced, substantially reduced, or eliminated by mutating the polymerase gene. Such mutations include point mutations, frame shift mutations, deletions and insertions. Preferably, the region of the gene encoding the 3'-to,-5' exonuclease activity is mutated or deleted using techniques well known in the art (Sambrook et al., (1989) in: Molecular Cloning, A Laboratory Manual (2nd Ed.), Cold Spring Harbor Laboratory Press, Cold Spring Harbor, NY).

The 3'-to-5' exonuclease activity can be reduced or impaired by creating site specific mutants within the 3'→5' exonuclease domain. *See infra.* In a specific embodiment of the invention Asp³²³ of *The* DNA polymerase is changed to any amino acid, preferably to Ala³²³ to substantially reduce 3'-to-5' exonuclease activity. In another specific embodiment of the invention, Asp³²³ of *Tma* may be changed to any other amino acid, preferably to Ala to substantially reduce 3'-to-5' exonuclease activity. The following represents a domain of interest for a number of polymerases for preparing 3'to 5'exonuclease mutants, where only Tne is of the invention

| | | | | |
|---|---|---|---|---|
| *Tne* | 318 | PSFALDLETSS | 328 | (SEQ ID NO:8) |
| PolI | 350 | PVFAFDTETDS | 360 | (SEQ ID NO:9) |
| T5 | 159 | GPVAFDSETSA, | 169 | (SEQ ID NO:10) |
| T7 | 1 | MIVSDIEANA | 10 | (SEQ ID NO: 11) |

Mutations, such as insertions, deletions and substitutions within the above domain can result in substantially reduced 3'-5' exonuclease activity. By way of example, Asp³⁵⁵ (polI), Asp¹⁶⁴ (T5), and Asp⁵ (T7) may be substituted with any amino acid to substantially reduce 3-5' exonuclease activity. For example, Asp at these positions may be substituted with Ala.

The 5'→3' exonuclease activity of the polymerases can be reduced or eliminated by mutating the polymerase gene or by deleting the 5' to 3' exonuclease domain. Such mutations include point mutations, frame shift mutations, deletions, and insertions. Preferably, the region of the gene encoding the 5'→3 ' exonuclease activity is deleted using techniques well known in the art. Any one of six conserved ammo acids that are associated with the 5'→3' exonuclease activity can be mutated. Examples of these conserved amino acids with respect to *Tne* DNA polymerase include Asp⁸, Glu¹¹², Asp¹¹⁴, Asp¹¹⁵ Asp¹³⁷, and Asp¹³⁹. Other possible sites for mutation are: Gly¹⁰², Gly¹⁸⁷ and Gly¹⁹⁵.

Corresponding amino acid to target for other polymerases not of the invention to reduce or eliminate 5'-3' exonuclease activity as follows:

| | |
|---|---|
| *E. coli* polI: | Asp¹³, Glu¹¹³, Asp¹¹⁵ Asp¹¹⁶, A¹³⁸ and Asp¹⁴⁰. |
| *Taq* pol: | Asp¹⁸, Glu¹¹⁷, Asp¹¹⁹, Asp¹²⁰, Asp¹⁴², and Asp¹⁴⁴, |
| *Tma* pol: | Asp⁸, Glu¹¹², Asp¹¹⁴, Asp¹¹⁵, Asp¹³⁷, and Asp¹³⁹ |

Amino acid residues of *Taq* DNA polymerase are as numbered in U.S. 5,079,352. Amino acid residues of *Thermotogamaritima (Tma)* DNApolymerase are numbered as in U.S. Patent No. 5,374,553.

Examples of other amino acids which may be targeted for other polymerases not being of the invention to reduce 5' to 3' exonuclease activity include:

| **Enzyme or source** | **Mutation positions** |
|---|---|
| *Streptococcus pneumoniae* | Asp¹⁰, Glu¹¹⁴, Asp¹¹⁶, Asp¹¹⁷, Asp¹³⁹, Asp¹⁴¹ |
| *Thermus flavus* | Asp¹⁷, Glu¹¹⁶, Asp¹¹⁸, Asp¹¹⁹, Asp¹⁴¹ Asp¹⁴³ |
| *Thermus thermophilus* | Asp¹⁸, Glu¹¹⁸, Asp¹²⁰ , Asp¹²¹ , Asp¹⁴³ Asp¹⁴⁵ |
| *Deinococcus radiodurans* | Asp¹⁸, Glu¹¹⁷, Asp¹¹⁹, Asp¹²⁰, Asp¹⁴² Asp¹⁴⁴ |
| *Bacillus caldotenax* | Asp⁹, Glu¹⁰⁹; Asp¹¹¹, Asp¹¹², Asp¹³⁴ , Asp¹³⁶ |

Coordinates of *S. pneumonia, T. flavus, D. radiodurans, B. caldotenax* were obtained from Gutman and Minton. Coordinates of *T. thermophilus* were obtained from International Patent No. WO 92/06200.

Polymerase mutants can also be made to render the polymerase non-discriminating against non-natural nucleotides such as dideoxynucleotides (see U.S.Patent 5,614,365).Changes within the O-helix, such as other point mutations, deletions, and insertions, can be made to render the polymerase non-discriminating. By way of example, one *Tne* DNA polymerase mutant having this property substitutes a nonnatural amino acid such as Tyr for Phe (F) in the O-helix.

Corresponding mutants for polymerases other than Tne of the invention can be made to increase nondiscrimination for ddNTPs. For example, mutants can also be prepared from *Tma* DNA polymerase at positions Lys⁷²⁶ and Phe⁷³⁰. Mutants include Phe⁷³⁰ to Tyr⁷³⁰, Ser⁷³⁰, Thr⁷³⁰ and Ala⁷³⁰. Likewise, PolI Lys⁷⁵⁸ and Phe⁷⁶²; *Taq* Lys⁶⁶³ and Phe⁶⁶⁷; and T7 Lys⁵²² and Phe⁵²⁶ can be mutated.

Typically, the 5'-3' exonuclease activity, 3' to 5' exonuclease activity, discriminatory activity and fidelity can be affected by substitution of amino acids typically which have different properties. For example, an acidic amino acid such as Asp may be changed to a basic, neutral or polar but uncharged amino acid such as Lys, Arg, His (basic); Ala, Val, Leu, Ile, Pro, Met, Phe, Trp (neutral); or Gly, Ser, Thr, Cys, Tyr, Asn or Gln (polar but uncharged). Glu may be changed to Asp, Ala, Val Leu, Ile, Pro, Met, Phe, Trp, Gly, Ser, Thr, Cys, Tyr, Asn or Gln.

Preferably, oligonucleotide directed mutagenesis is used to create the mutant polymerases which allows for all possible classes of base pair changes at any determined site along the encoding DNA molecule. In general, this technique involves annealing a oligonucleotide complementary (except for one or more mismatches) to a single stranded nucleotide sequence coding for the DNA polymerase of interest. The mismatched oligonucleotide is then extended by DNA polymerase, generating a double stranded DNA molecule which contains the desired change in sequence on one strand. The changes in sequence can of course result in the deletion, substitution, or insertion of an amino acid. The double stranded polynucleotide can then be inserted into an appropriate expression vector, and a mutant polypeptide can thus be produced. The above-described oligonucleotide directed mutagenesis can of course be carried out via PCR.

### Enhancing Expression of Polymerases

To optimize expression of the polymerases of the present invention, inducible or constitutive promoters are well known and may be used to express high levels of a polymerase structural gene in a recombinant host. Similarly, high copy number vectors, well known in the art, may be used to achieve high levels of expression. Vectors having an inducible high copy number may also be useful to enhance expression of the polymerases of the invention in a recombinant host.

To express the desired structural gene in a prokaryotic cell (such as, *E. coli, B. subtilis, Pseudomonas,* etc.), it is necessary to operably link the desired structural gene to a functional prokaryotic promoter. However, the natural promoter of the polymerase gene may function in prokaryotic hosts allowing expression of the polymerase gene. Thus, the natural promoter or other promoters may be used to express the polymerase gene. Such other promoters may be used to enhance expression and may either be constitutive or regulatable (i.e., inducible or derepressible) promoters. Examples of constitutive promoters include the *int* promoter of bacteriophage λ, and the *bla* promoter of the β-lactamase gene of pBR322. Examples of inducible prokaryotic promoters include the major right and left promoters of bacteriophage λ (P_{R} and P_{L})*, trp, rec*A*, lac*Z*, lac*I*, tet, gal, trc,* and *tac* promoters of *E. coli.* The *B. subtilis* promoters include α-amylase (Ulmanen et al., J. Bacteriol 162:176-182 (1985)) and *Bacillus* bacteriophage promoters (Gryczan, T., In: The Molecular Biology Of Bacilli, Academic Press, New York (1982)). *Streptomyces* promoters are described by Ward et al., Mol. Gen. Genet. 203:468478 (1986)). Prokaryotic promoters are also reviewed by Glick, J. Ind Microbiol. 1:277-282 (1987); Cenatiempto, Y., Biochimie 68:505-516 (1986); and Gottesman, Ann. Rev. Genet. 18:415-442 (1984). Expression in a prokaryotic cell also requires the presence of a ribosomal binding site upstream ofthe gene-encoding sequence. Such ribosomal binding sites are disclosed, for example, by Gold et al., Ann. Rev. Microbiol. 35:365404 (1981).

To enhance the expression of polymerases of the invention in a eukaryotic cell, well known eukaryotic promoters and hosts may be used. Preferably, however, enhanced expression of the polymerases is accomplished in a prokaryotic host. The preferred prokaryotic host for overexpressing this enzyme is *E. coli.*

### Isolation and Purification of Polymerases

The enzyme(s) of the present invention is preferably produced by fermentation of the recombinant host containing and expressing the desired DNA polymerase gene. However, the DNA polymerases of the present invention may be isolated from any strain which produces the polymerase of the present invention. Fragments of the polymerase are also included in the present disclosure Such fragments include proteolytic fragments and fragments having polymerase activity.

Any nutrient that can be assimilated by a host containing the cloned polymerase gene may be added to the culture medium. Optimal culture conditions should be selected case by case according to the strain used and the composition of the culture medium. Antibiotics may also be added to the growth media to insure maintenance of vector DNA containing the desired gene to be expressed. Media formulations have been described in DSM or ATCC Catalogs and Sambrook et al., In: Molecular Cloning, a Laboratory Manual (2nd ed.), Cold Spring Harbor Laboratory Press, Cold Spring Harbor, NY (1989).

Recombinant host cells producing the polymerases of this invention can be separated from liquid culture, for example, by centrifugation. In general, the collected microbial cells are dispersed in a suitable buffer, and then broken down by ultrasonic treatment or by other well known procedures to allow extraction of the enzymes by the buffer solution. After removal of cell debris by ultracentrifugation or centrifugation, the polymerase can be purified by standard protein purification techniques such as extraction, precipitation, chromatography, affinity chromatography, electrophoresis or the like. Assays to detect the presence of the polymerase during purification are well known in the art and can be used during conventional biochemical purification methods to determine the presence of these enzymes.

### Uses of Polymerases

The polymerases of the present invention may be used in well known nucleic acid synthesis, sequencing, labeling, amplification or cDNA synthesis reactions.

As is well known, sequencing reactions (isothermal DNA sequencing and cycle sequencing of DNA) require the use of polymerases. Dideoxy-mediated sequencing involves the use of a chain-termination technique which uses a specific polymer for extension by DNA polymerase, a base-specific chain terminator and the use of polyacrylamide gels to separate the newly synthesized chain-terminated DNA molecules by size so that at least a part of the nucleotide sequence of the original DNA molecule can be determined. Specifically, a DNA molecule is sequenced by using four separate DNA sequence reactions, each of which contains different base-specific terminators (or one reaction iffluorescent terminators are used). For example, the first reaction will contain a G-specific terminator, the second reaction will contain a T-specific terminator, the third reaction will contain an A-specific terminator, and a fourth reaction may contain a C-specific terminator. Preferred terminator nucleotides include dideoxyribonucleoside triphosphates (ddNTPs) such as ddATP, ddTTP, ddGTP, ddITP and ddCTP. Analogs of dideoxyribonucleoside triphosphates may also be used and are well known in the art.

When sequencing a DNA molecule, ddNTPs lack a hydroxyl residue at the 3' position of the deoxyribose base and thus, although they can be incorporated by DNA polymerases into the growing DNA chain, the absence of the 3'-hydroxy residue prevents formation of the next phosphodiester bond resulting in termination of extension of the DNA molecule. Thus, when a small amount of one ddNTP is included in a sequencing reaction mixture, there is competition between extension of the chain and base-specific termination resulting in a population of synthesized DNA molecules which are shorter in length than the DNA template to be sequenced. By using four different ddNTPs in four separate enzymatic reactions, populations of the synthesized DNA molecules can be separated by size so that at least a part of the nucleotide sequence of the original DNA molecule can be determined. DNA sequencing by dideoxy-nucleotides is well known and is described by Sambrook et al., In: Molecular Cloning, a Laboratory Manual, Cold Spring Harbor Laboratory Press, Cold Spring Harbor, NY (1989). As will be readily recognized, the polymerases of the present invention may be used in such sequencing reactions.

As is well known, detectably labeled nucleotides are typically included in sequencing reactions. Any number of labeled nucleotides can be used in sequencing (or labeling) reactions, including, but not limited to, radioactive isotopes, fluorescent labels, chemiluminescent labels, bioluminescent labels, and enzyme labels. For example the polymerases of the present invention may be useful for incorporating αS nucleotides ([αS]dATP, [αS]dTTP, [αS]dCTP and [αS]dGTP) during sequencing (or labeling) reactions.

Polymerase chain reaction (PCR), a well known DNA amplification technique, is a process by which DNA polymerase and deoxyribonucleoside triphosphates are used to amplify a target DNA template. In such PCR reactions, two primers, one complementary to the 3' termini (or near the 3'-termini) of the first strand of the DNA molecule to be amplified, and a second primer complementary to the 3' termini (or near the 3'-termini) of the second strand of the DNA molecule to be amplified, are hybridized to their respective DNA strands. After hybridization, DNA polymerase, in the presence of deoxyribonucleoside triphosphates, allows the synthesis of a third DNA molecule complementary to the first strand and a fourth DNA molecule complementary to the second strand of the DNA molecule to be amplified. This synthesis results in two double stranded DNA molecules. Such double stranded DNA molecules may then be used as DNA templates for synthesis of additional DNA molecules by providing a DNA polymerase, primers, and deoxyribonucleoside triphosphates. As is well known, the additional synthesis is carried out by "cycling" the original reaction (with excess primers and deoxyribonucleoside triphosphates) allowing multiple denaturing and synthesis steps. Typically, denaturing of double stranded DNA molecules to form single stranded DNA templates is accomplished by high temperatures. The DNA polymerases of the present invention are heat stable DNA polymerases, and thus will survive such thermal cycling during DNA amplification reactions. Thus, the DNA polymerases of the invention are ideally suited for PCR reactions, particularly where high temperatures are used to denature the DNA molecules during amplification.

The DNA polymerase mutants of the present invention may also be used to prepare cDNA from RNA templates. See, U. S. Patent Nos. 5,405,776 and 5,244,797. Thus, the invention also relates to a method of preparing one or more cDNA molecules from one or more mRNA templates, comprising
(a) contacting one or more mRNA templates with one or more oligo(dT) primers or other complementary primers to form one or more hybrids, and
(b) contacting said one or more hybrids with one or more of the DNA polymerase mutants of the invention and with the four dNTPs, whereby one or more cDNA-RNA hybrids are obtained.

If the reaction mixture in step (b) further comprises an appropriate oligonucleotide which is complementary to the cDNA being produced, it is also possible to obtain dsDNA following first strand synthesis. Thus, the invention is also directed to a method of preparing double stranded cDNA with the DNA polymerases of the present invention.

### Kits

The polymerases of the invention are suited for the preparation of kits. Kits comprising the polymerase(s) may be used for detectably labeling molecules, sequencing, amplifying and synthesizing molecules or cDNA synthesis by well known techniques, depending on the content of the kit. See U.S. Patent Nos. 4,962,020, 5,173,411, 4,795,699, 5,498,523, 5,405,776 and 5,244,797. Such kits may comprise a carrier being compartmentalized to receive in close confinement one or more containers such as vials, test tubes and the like. Each of such containers may contain one or more components or mixtures of components needed to perform nucleic acid synthesis, sequencing, labeling, amplification, or cDNA synthesis.

Kits for sequencing DNA may comprise one or more containers. A first container may contain, for example, one or more substantially purified DNA polymerases of the invention (or mutants, fragments, variants or derivatives thereof). Additional containers of such kits may contain, for example, one or more nucleotides needed to synthesize a DNA molecule complementary to DNA template, one or more nucleic acid synthesis terminating agents (such one or more dideoxynucleoside triphosphates), pyrophosphatase, one or more primers, and/or one or more suitable sequencing buffers.

Kits used for amplifying or synthesis of nucleic acids may comprise one or more containers. A first container may contain, for example, one or more substantially pure polymerases of the invention, or mutants, variants, fragments or derivatives thereof Additional containers of such kits may contain, for example, one or more nucleotides or mixtures of nucleotides, one or mor primers, and/or one or more suitable amplification or synthesis buffers.

Kits for cDNA synthesis may comprise one or more containers. A first container, for example, may contain one or more substantially purified polymerases of the invention, or mutants, variants, fragments or derivatives thereof. Additional containers in such kits may contain, for example, one or more dNTPs, and/or one or more primers such as one or more oligo(dT) primers. See U.S. Patent Nos. 5,405,776 and 5,244,797. Since the DNA polymerases of the present invention are also capable of preparing dsDNA, kits according to this aspect ofthe invention may comprise one or more additional containers containing one or more appropriate primers complementary to the first strand cDNA.

When desired, the kits of the present invention may optionally comprise one or more containers which contain one or more detectably labeled nucleotides-which may be used during the synthesis or sequencing of nucleic acid molecules. Any of a number of labels may be used to detect such nucleotides, including, but not limited to, radioactive isotopes, fluorescent labels, chemiluminescent labels, bioluminescent labels and enzyme labels.

Having now described the present invention in detail, the same will be more clearly understood by reference to the following examples, which are included herewith for purposes of illustration only and are not intended to be limiting of the invention.

### Example 1: Bacterial Strains And Growth Conditions

*Thermotoga neapolitana* DSM No. 5068 was grown under anaerobic conditions as described in the DSM catalog (addition of resazurin, Na₂S, and sulfur granules while sparging the media with nitrogen) at 85°C in an oil bath from 12 to 24 hours. The cells were harvested by filtering the broth through Whatman #1 filter paper. The supernatant was collected in an ice bath and then centrifuged in a refrigerated centrifuge at 8,000 rpms for twenty minutes. The cell paste was stored at -70°C prior to total genomic DNA isolation.

*E. coli* strains were grown in 2X LB broth base (Lennox L broth base: GIBCO/BRL) medium. Transformed cells were incubated in SOC (2% tryptone, 0.5% yeast extract, yeast 10 mM NaCl, 2.5 mM KCl, 20mM glucose, 10mM MgCl₂, and 10mM MgSO₄ per liter) before plating. When appropriate antibiotic supplements were 20 mg/l tetracycline and 100 mg/l ampicillin. *E. coli* strain DH10B (Lorow et al., Focus 12:19-20 (1990)) was used as host strain. Competent DH10B may be obtained from Life Technologies, Inc. (LTI) (Rockville, MD).

### Example 2: DNA Isolation

*Thermotoga neapolitana* chromosomal DNA was isolated from 1.1 g of cells by suspending the cells in 2.5 ml TNE (50mM Tris-HCl, pH 8.0, 50mM NaCl, 10mM EDTA) and treated with 1% SDS for 10 minutes at 37°C. DNA was extracted with phenol by gently rocking the lysed cells overnight at 4°C. The next day, the lysed cells were extracted with chloroform:isoamyl alcohol. The resulting chromosomal DNA was further purified by centrifugation in a CsCl density gradient. Chromosomal DNA isolated from the density gradient was extracted three times with isopropanol and dialyzed overnight against a buffer containing 10 mM Tris-HCl (pH 8.0) and 1 mM EDTA (TE).

### Example 3: Construction of Genomic Libraries

The chromosomal DNA isolated in Example 2 was used to construct a genomic library in the plasmid pCP13. Briefly, 10 tubes each containing 10µg of *Thermotoga neapolitana* chromosomal DNA was digested with 0.01 to 10 units of Sau3A1 for 1 hour at 37°C. A portion of the digested DNA was tested in an agarose (1.2%) gel to determine the extent of digestion. Samples with less than 50% digestion were pooled, ethanol precipitated and dissolved in TE. 6.5 µg of partially digested chromosomal DNA was ligated into 1.5 µg of pCP 13 cosmid which had been digested with *Bam*HI restriction endonuclease and dephosphorylated with calf intestinal alkaline phosphatase. Ligation of the partially digested *Thermotoga* DNA and *Bam*HI cleaved pCP13 was carried out with T4 DNA ligase at 22°C for 16 hours. After ligation, about 1µg of ligated DNA was packaged using λ-packaging extract (obtained from Life Technologies, Inc., Rockville, MD). DH10B cells (Life Tech, Inc.) were then infected with 100 µl of the packaged material. The infected cells were plated on tetracycline containing plates. Serial dilutions were made so that approximately 200 to 300 tetracycline resistant colonies were obtained, per plate.

### Example 4: Screening for Clones Expressing Thermotoga neapolitana DNA Polymerase

Identification ofthe *Thermotoganeapolitana* DNA polymerase gene of the invention was cloned using the method of Sagner et al., Gene 97:119-123 (1991). Briefly, the *E. coli* tetracycline resistant colonies from Example 3 were transferred to nitrocellulose membranes and allowed to grow for 12 hours. The cells were then lysed with the fumes of chloroform:toluene (1:1) for 20 minutes and dried for 10 minutes at room temperature. The membranes were then treated at 95°C for 5 minutes to inactivate the endogenous *E. coli* enzymes. Surviving DNA polymerase activity was detected by submerging the membranes in 15 ml of polymerase reaction mix (50 mM Tris-HCl (pH 8.8), 1 mM MgCl₂, 3 mM β-mercaptoethanol, 10 µM dCTP, dGTP, dTTP, and 15 µCi of 3,000 Ci/mmol [α³²P]dATP) for 30 minutes at 65°C.

Using autoradiography, three colonies were identified that expressed a *Thermotoga neapolitana DNA* polymerase. The cells were grown in liquid culture and the protein extract was made by sonication. The presence of the cloned thermostable polymerase was confirmed by treatment at 90°C followed by measurement of DNA polymerase activity at 72°C by incorporation of radioactive deoxyribonucleoside triphosphates into acid insoluble DNA. One of the clones, expressing *Tne* DNA polymerase, contained a plasmid designated pCP 13-32 and was used for further study.

### Example 5: Subcloning of Tne DNA polymerase

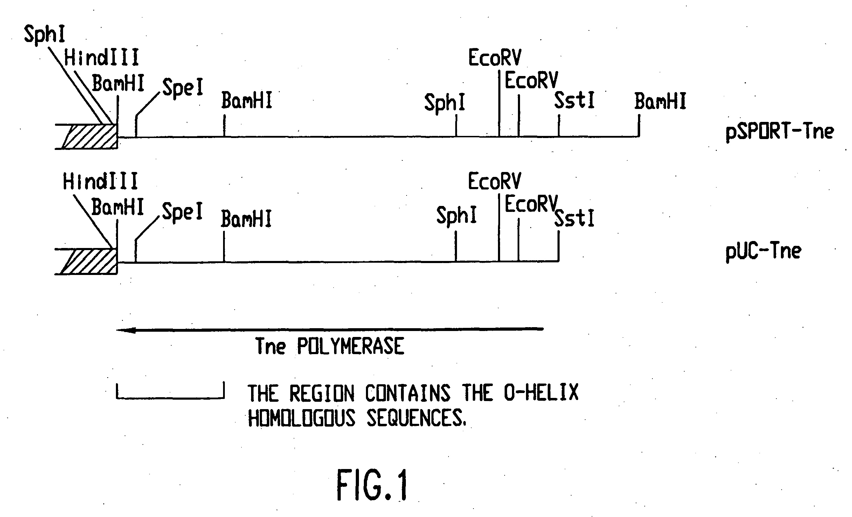

Since the pCP 13-32 clone expressing the *Tne* DNA polymerase gene contains about 25 kb of *T*. *neapolitana* DNA, subcloning a smaller fragment of the *Tne* polymerase gene was attempted. The molecular weight of the *Tne* DNA polymerase purified from *E. coli*/*pCP*13-32 was about *100* kD. Therefore, a 2.5-3.0 kb DNA fragment will be sufficient to code for full-length polymerase. A second round of *Sau3A* partial digestion similar to Example 3 was done using pCP13-32 DNA. In this case, a 3.5 kb region was cut out from the agarose gel, purified by Gene Clean-(BIO 101, La Jolla, CA) and ligated into plasmid pSport 1 (Life Technologies, Inc.) which had been linearized with *Bam*HI *,* and dephosphorylated with calfintestinal alkatine phosphatase. After ligation, DH10B was transformed and colonies were tested for DNA polymerase activity as described in Example 1. Several clones were identified that expressed *Tne* DNA polymerase. One of the clones (pSport-Tne) containing about 3 kb insert was further characterized. A restriction map of the DNA fragment is shown in Fig. 1. Further, a 2.7 Kb *Hind*III*-Sst*I fragment was subcloned into pUC19 to generate pUC19-Tne. *E. coli*/pUC19-*Tne* also produced Tn DNA polymerase. This plasmid was deposited under the identification reference *E. coli* DH10B (pUC-*Tne)* on September 30, 1994, with the Collection, Agricultural Research Culture Collection (NRRL), 1815 Peoria, IL 61604 as Deposit No. NRRL B-21338. The nucleotide and amino acid sequence of *Tne* polymerase is described in the art.

### Example 6: Purification of Thermotoga neapolitana DNA Polymerase from E. coli

Twelve grams of *E. coli* cells expressing cloned *Tne* DNA polymerase (DH10B/pSport-Tne) were lysed by sonication (four thirty-second bursts with a medium tip at the setting of nine with a Heat Systems Ultrasonics Inc., model 375 sonicator) in 20 ml of ice cold extraction buffer (50 mM Tris HCl (pH 7.4), 8% glycerol, 5 mM mercaptoethanol, 10 mum NaCl, 1 mM EDTA, 0.5 mM PMSF). The sonicated extract was heated at 80°C for 15 min. and then cooled in ice for 5 min. 50 mM KCl and PEI (0.4%) was added to remove nucleic acids. The extract was centrifuged for clarification. Ammonium sulfate was added to 60%, the pellet was collected by centrifugation and resuspended in 10 ml of column buffer (25 mM Tris-HCl (pH 7.4), 8% glycerol, 0.5% EDTA, 5M 2-mercaptoethanol, 10 mM KCl). A Blue-Sepharose (Pharmacia) column, or preferably a Toso heparin (TosoHaas) column, was washed with 7 column volumes of column buffer and eluted with a 15 column volume gradient of buffer from 10mM to 2 M KCl. Fractions containing polymerase activity were pooled. The fractions were dialyzed against 20 volumes of column buffer. The pooled fractions were applied to a Toso650Q column (TosoHaas). The column was washed to baseline OD₂₈₀ and elution effected with a linear 10 column volume gradient of 25 mM Tris (pH 7.4), 8% glycerol, 0.5 mM EDTA, 10 mM KCl, 5 mM β-mercaptoethanol to the same buffer plus 650 mM KCl. Active fractions were pooled.

### Example 7: Construction of Thermotoga neapolitana 3'-to-5' Exonuclease Mutant

The amino acid sequence of portions of the *Tne* DNA polymerase was compared with other known DNA polymerases such as *E. coli* DNA polymerase 1, *Taq* DNA polymerase, T5 DNA polymerase, and T7 DNA polymerase to localize the regions of 3'-to-5' exonuclease activity, and the dNTP binding domains within the DNA polymerase. One of the 3'-to-5' exonuclease domains was determined based on the comparison of the amino acid sequences of various DNA polymerases (Blanco, L., et al. Gene 112: 139-144 (1992); Braithwaite and Ito, Nucleic Acids Res. 21: 787-802 (1993)) is as follows:

| | | | | |
|---|---|---|---|---|
| *Tne* | 318 | PSFALDLETSS | 328 | (SEQ ID NO:8) |
| PolI | 350 | PVFAFDTETDS | 360 | (SEQ ID NO:9) |
| T5 | 159 | GPVAFDSETSA | 169 | (SEQ ID NO: 10) |
| T7 | 1 | MIVSDIEANA | 10 | (SEQ ID NO:11) |

As a first step to make the *Tne* DNA polymerase devoid of- 3'→5' exonuclease activity, a 2kb *Sph* fragment from pSport-Tne was cloned into M13mp19 (LTI, Rockville, MD). The recombinant clone was selected in *E. coli* DHSαF'IQ (LTI, Rockville, MD). One of the clones with the proper insert was used to isolate uracilated single-stranded DNA by infecting *E*. *coli* CJ236 (BioRad; Hercules, California) with the phage particle obtained from *E*. *coli* DHSαF'IQ. An oligonucleotide, GA CGT TTC AAG CGC TAG GGC AAA AGA (SEQ ID NO:12) was used to perform site directed mutagenesis. This site-directed mutagenesis converted Asp³²³ (indicated as * above) to Ala³²³. An *Eco*47III restriction site was created as part of this mutagenesis to facilitate screening of the mutant following mutagenesis. The mutagenesis was performed using a protocol as described in the BioRad manual (1987) except T7 DNA polymerase was used instead of T4 DNA polymerase (U.S. Biochemicals; Cleveland, OH). The mutant clones were screened for the Eco47III restriction site that was created in the mutagenic oligonucleotide. One of the mutants having the created Eco47III restriction site was used for further study. The mutation Asp³²³ to Ala³²³ has been confirmed by DNA sequencing.

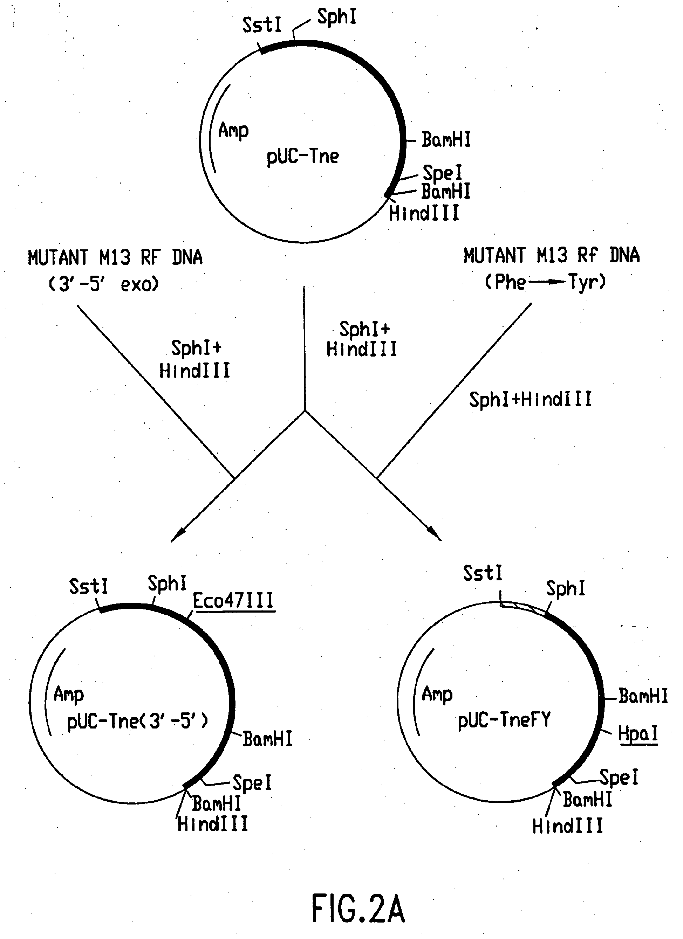

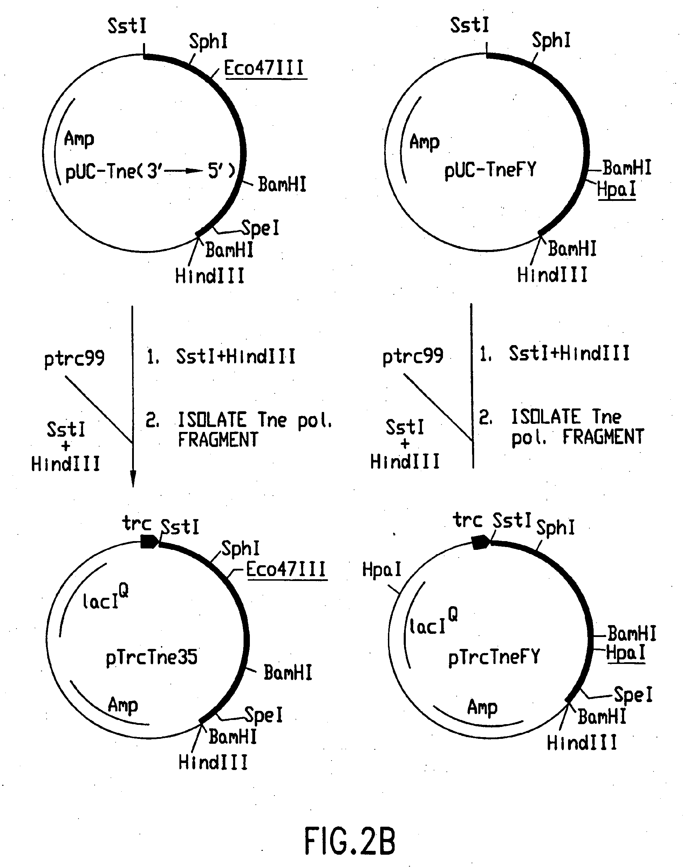

To incorporate the 3'-to-5' exonuclease mutation in an expression vector, the mutant phage was digested with *Sph*I and *Hind*III*.* A 2 kb fragment containing the mutation was isolated. This fragment was cloned in pUC-Tne to replace the wild type fragment. See Figure 2A. The desired clone, pUC-Tne (3'→5'), was isolated. The presence of the mutant sequence was confirmed by the presence of the unique *Eco47*III site. The plasmid was then digested with *Sst*I and *Hind*III. The entire mutant polymerase gene (2.6 kb) was purified and cloned into *Sst*I and *Hind*III digested pTrc99 expression vector (Pharmacia, Sweden). The clones were selected in DH10B (LTI, Rockville, MD). The resulting plasmid was designated pTrcTne35. See Figure 2B. This clone produced active heat stable DNA polymerase.

### Example 8: Phenylalanine to Tyrosine Mutant

As discussed above, the polymerase active site including the dNTP binding domain is usually present at the carboxyl terminal region of the polymerase. The sequence of the *Tne* polymerase gene suggests that the amino acids that presumably contact and interact with the dNTPs are present within the 694 bases starting at the internal *Bam*HI site. *See* Figure 1. This conclusion is based on homology with a prototype polymerase *E. coli* DNA polymerase 1. *See* Polisky et al., J. Biol. Chem. 265:14579-14591 (1990). A comparison was made of the O-helix for various polymerases:

| | | | | |
|---|---|---|---|---|
| *Tne* | 722 | RRVGKMVNFSIIYG | 735 | (SEQ ID NO:13) |
| Pol I | 754 | RRSAKAINFGLIYG | 767 | (SEQ ID NO:2) |
| T5 | 562 | RQAAKAITFGILYG | 575 | (SEQ ID NO:6) |
| T7 | 518 | RDNAKTFIYGFLYG | 531 | (SEQ ID NO:4) |
| *Taq* | 659 | RRAAKTINFGVLYG | 672 | (SEQ ID NO:3) |

It has been shown that by replacing the phenylalanine residue of *Taq* DNA polymerase, the polymerase becomes non-discriminating against non-natural nucleotides such as dideoxynucleotides. *See* U.S. Patent No. 5,614,356. The mutation was based on the assumption that T7 DNA polymerase contains a tyrosine residue in place of the phenylalanine, and T7 DNA polymerase is non-discriminating against dideoxynucleotides. The corresponding residue, Phe⁷⁶² of *E. coli* PolI is an amino acid that directly interacts with nucleotides. (Joyce and Steitz, Ann. Rev. Biochem. 63:777-822 (1994); Astake, M.J., J. Biol. Chem. 270:1945-1954 (1995)). A similar mutant of *Tne* DNA polymerase was prepared.

In order to change Phe⁷³⁰ of the *Tne* polymerase to a Tyr⁷³⁰ site directed mutagenesis was performed using the oligonucleotide GTA TAT TAT AGA GTA GTT AAC CAT CTT TCC A (SEQ ID NO:14). As part of this oligonucleotide directed mutagenesis, a *Hpa*I restriction site was created in order to screen mutants easily. The same uracilated single-stranded DNA and mutagenesis procedure described in Example 7 were used for this mutagenesis. Following mutagenesis, the mutants were screened for the *Hpa*I site. Mutants with the desired *Hpa*I site were used for further study. The mutation has been confirmed by DNA sequencing.

The Phe⁷³⁰ to Tyr⁷³⁰ mutation was incorporated into pUC-*Tne* by replacing the wild type *Sph*I*-Hind*III fragment with the mutant fragment obtained from the mutant phage DNA. The presence of the desired clone, pUC-TneFY, was confirmed by the presence of the unique *Hpa*I site, see Figure 2A. The entire mutant polymerase gene was subcloned into pTrc99 as an *Sst*I*-Hind*III fragment as described above in DH10B. The resulting plasmid was designated pTrcTneFY. (Figure 2B). The clone produced active heat stable polymerase.

### Example 9: 3'-to-5' Exonuclease and Phe⁷³⁰→Tyr⁷³⁰ Double Mutants

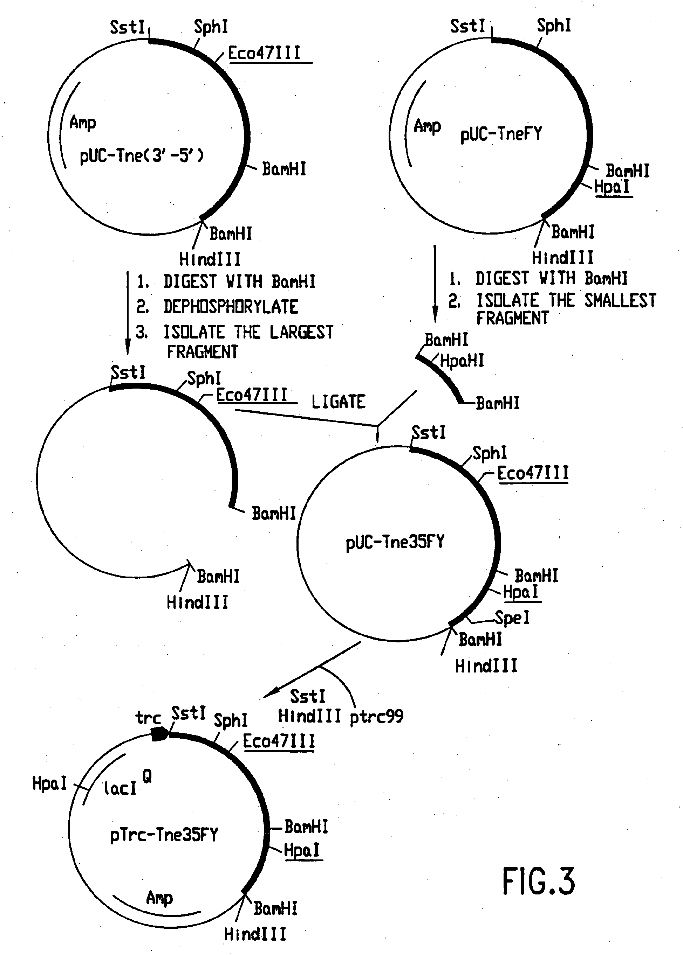

In order to introduce the 3'→5' exonuclease mutation and the Phe⁷³⁰→Tyr⁷³⁰ mutation in the same expression vector, pTrc99, it was necessary to first reconstitute both mutations in the pUC-Tne clone. See Figure 3. Both the pUC-Tne (3'→5') and the pUC-TneFY were digested with *Bam*HI. The digested pUC-Tne (3'→5') was dephosphorylated to avoid recirculation in the following ligations. The resulting fragments were purified on a 1% agarose gel. The largest *Bam*HI fragment (4.4 kb) was purified from pUC-Tne (3'→5') digested DNA and the smallest *Bam*HI fragment (0.8 kb) containing the Phe⁷³⁰→Tyr⁷³⁰ mutation was purified and ligated to generate pUC-Tne35FY. The proper orientation and the presence of both mutations in the same plasmid was confirmed by *Eco*47III, *Hpa*I*,* and *Sph*I*-Hind*III restriction digests. See Figure 3.

The entire polymerase containing both mutations was subcloned as a *Sst*I-HindIII fragment in pTrc99 to generate pTrcTne35FY in DH10B. The clone produced active heat stable polymerase.

### Example 10: 3'-to-5' Exonuclease, 5'-to-3' Exonuclease, and Phe⁷³⁰→Tyr⁷³⁰ Triple Mutants

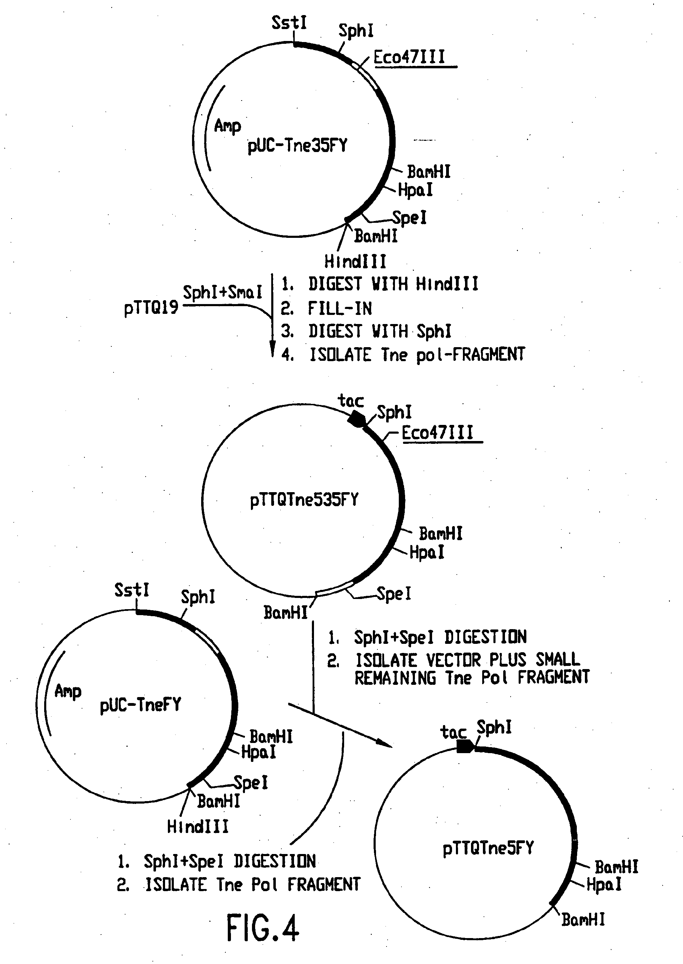

In most of the known polymerases, the 5'-to-3' exonuclease activity is present at the amino terminal region of the polymerase (Ollis, D.L., et al., Nature 313, 762-766, 1985; Freemont, P.S., et al., Proteins 1: 66-73, 1986; Joyce, C.M., Curr. Opin. Struct. Biol. 1: 123-129 (1991). There are some conserved amino acids that are implicated to be responsible for 5'-to-3' exonuclease activity (Gutman and Minton, Nucl. Acids Res. 21: 4406-4407, 1993). *See supra.* It is known that 5'-to-3' exonuclease domain is dispensable. The best known example is the Klenow fragment of *E. coli* Pol I. The Klenow fragment is a natural proteolytic fragment devoid of 5'-to-3' exonuclease activity (Joyce, C.M., et al., J. Biol. Chem. 257: 1958-1964, 1990). In order to generate an equivalent mutant for *Tne* DNA polymerase devoid of 5'-to-3' exonuclease activity, the presence of a unique *Sph*I site present 680 bases from the *Sst*I site was exploited. pUC-Tne35FY was digested with *Hind*III, filled-in with Klenow fragment to generate a blunt-end, and digested with *Sph*I. The 1.9 kb fragment was cloned into an expression vector pTTQ19 (Stark, M.J.R., Gene 51: 255-267, 1987) at the *Sph*I*-Sma*I sites and was introduced into DH10B. This cloning strategy generated an in-frame polymerase clone with an initiation codon for methionine from the vector. The resulting clone is devoid of 219 amino terminal amino acids of *Tne* DNA polymerase. This clone is designated as pTTQTne535FY (Fig. 4). The clone produced active heat stable polymerase. No exonuclease activity could be detected in the mutant polymerase as evidenced by lack of presence of unusual sequence ladders in the sequencing reaction. This particular mutant polymerase is highly suitable for DNA sequencing.

### Example 11: 5'-to-3' Exonuclease Deletion and Phe⁷³⁰→Tyr⁷³⁰ Substitution Mutant

In order to generate the 5'→3' exonuclease deletion mutant of the *Tne* DNA polymerase Phe⁷³⁰→Tyr⁷³⁰ mutant, the 1.8 kb *Sph*I*-Spe*I fragment of pTTQTne535FY was replaced with the identical fragment of pUC-Tne FY. See Fig. 4. A resulting clone, pTTQTne5FY, produced active heat stable DNA polymerase. As measured by the rate of degradation of a labeled primer, this mutant has a modulated, low but detectable, 3'→5' exonuclease activity compared to wild type Tne DNA polymerase. M13/pUC Forward 23-Base Sequencing Primer™, obtainable from LTI, Rockville, MD, was labeled at the 5' end with [P³²] ATP and T4 kinase, also obtainable from LTI, Rockville, MD, as described by the manufacturer. The reaction mixtures contained 20 units of either wild-type or mutant *Tne* DNA polymerase, 0.25 pmol of labeled primer, 20 mM tricine, pH 8.7, 85 mM potassium acetate, 1.2 mM magnesium acetate, and 8% glycerol. Incubation was carried out at 70°C. At various time points, 10 µl aliquots were removed to 5 µl cycle sequencing stop solution and were resolved in a 6 % polyacrylamide sequencing gel followed by autoradiography. While the wild-type polymerase degraded the primer in 5 to 15 minutes, it took the mutant polymerase more than 60 minutes for the same amount of degradation of the primer. Preliminary results suggest that this mutant polymerase is able to amplify more than 12 kb of genomic DNA when used in conjunction with *Taq* DNA polymerase. Thus, the mutant polymerase is suitable for large fragment PCR.

### Example 12: Purification of the Mutant Polymerases

The purification of the mutant polymerases was done essentially as described Example 6, *supra,* with minor modifications. Specifically, 5 to 10 grams of cells expressing cloned mutant *Tne* DNA polymerase were lysed by sonication with a Heat Systems Ultrasonic, Inc. Model 375 machine in a sonication buffer comprising 50 mM Tris-HCl (pH 7.4); 8% glycerol; 5 mM 2-mercaptoethanol, 10 mM NaCl, 1 mM EDTA, and 0.5 mM PMSF. The sonication sample was heated at 75°C for 15 minutes. Following heat treatment, 200 mM NaCl and 0.4% PEI was added to remove nucleic acids. The extract was centrifuged for clarification. Ammonium sulfate was added to 48%, the pellet was resuspended in a column buffer consisting of 25 mM Tris-HCl (pH 7.4); 8% glycerol; 0.5% EDTA; 5 mM 2-mercaptoethanol; 10 mM KCl and loaded on a heparin agarose (LTI) column. The column was washed with 10 column volumes using the loading buffer and eluted with a 10 column volume buffer gradient from 10 mM to 1 M KCl. Fractions containing polymerase activity were pooled and dialyzed in column buffer as above with the pH adjusted to 7.8. The dialyzed pool of fractions were loaded onto a MonoQ (Pharmacia) column. The column was washed and eluted as described above for the heparin column. The active fractions are pooled and a unit assay was performed.

The unit assay reaction mixture contained 25 mM TAPS (pH 9.3), 2 mM MgCl₂, 50 mM KCl, 1 mM DTT, 0.2 mM dNTPs, 500 µg/ml DNase I-treated salmon sperm DNA, 21 mCi/ml [αP³²] dCTP and various amounts of polymerase in a final volume of 50 µl. After 10 minutes incubation at 70°C, 10 µl of 0.5 M EDTA was added to the tube. TCA-precipitable counts were measured in GF/C filters using 40 µl of the reaction mixture.

### Example 13: Generation of 5'-3'exonuclease mutant of full length Tne DNA polymerase

### 1. Identification of Two Amino Acids Responsible for 5'-3' Exonuclease Activity

*Tne* DNA polymerase contains three enzymatic activities similar to *E. coli* DNA polymerase I: 5'-3' DNA polymerase activity, 3'-5' exonuclease activity and 5'-3' exonuclease activity. This example is directed to the elimination of the 5'-3' exonuclease activity in full length *Tne* DNA polymerase. Gutman and Minton (Nucleic Acids Res. 21: 4406-4407 (1993)) identified six (A-F) conserved 5'-3' exonuclease domains containing a total of 10 carboxylates in various DNA polymerases in the polI family. Seven out of 10 carboxylates (in domains A, D and E) have been implicated to be involved in divalent metal ions binding as judged from the crystal structure (Kim et al. Nature 376: 612-616 (1995)) of *Taq* DNA polymerase. However, there was no clear demonstration that these carboxylates are actually involved 5'-3' exonuclease activity. In order to find out the biochemical characteristics of some of these carboxylates, two of the aspartic acids in domains A and E were chosen for mutagenesis. The following aspartic acids in these two domains were identified:

| | |
|---|---|
| *Tne* DNA polymerase: | 5 F L F **D⁸** G T 10 (domain A) (SEQ ID NO:15) |
| *Taq* DNA polymerase: | 15 L L V **D¹⁸** G H 20 (SEQ ID NO:16) |
| and | |
| *Tne* DNA polymerase: | 132 S L I T G **D¹³⁷** K D M L 141 (domain E) (SEQ ID NO:17) |
| *Taq* DNA polymerase: | 137 R I L T A **D¹⁴²** K D L Y 146 (SEQ ID NO:18) |

### 2. Isolation of Single Stranded DNA for Mutagenesis

Single stranded DNA was isolated from pSportTne (see *infra*)*.* pSportTne was introduced into DH5αF'IQ (LTI, Rockville, MD) by transformation. A single colony was grown in 2 ml Circle Grow (Bio 101, CA) medium with ampicillin at 37°C for 16 hrs. A 10 ml fresh media was inoculated with 0.1 ml of the culture and grown at 37°C until the A590 reached approximately 0.5. At that time, 0.1 ml of M13KO7 helper phage (1X10¹¹ pfu/ml, LTI) was added to the culture. The infected culture was grown for 75 min. Kanamycin was then added at 50 µg/ml, and the culture was grown overnight (16 hrs.). The culture was spun down. 9 ml of the supernatant was treated with 50 µg each of RNaseA and DNaseI in the presence of 10 mM MgCl₂ for 30 min. at room temperature. To this mixture, 0.25 volume of a cocktail of 3M ammonium acetate plus 20% polyethylene glycol was added and incubated for 20 min. on ice to precipitate phage. The phage was recovered by centrifugation. The phage pellet was dissolved in 200 µl of TE (10 mM Tris-HCl (pH 8) and 1 mM EDTA). The phage solution was extracted twice with equal volume of buffer saturated phenol (LTI, Rockville, MD), twice with equal volume of phenol: chloroform: isoamyl alcohol mixture (25:24: 1, LTI, Rockville, MD) and finally, twice with chloroform: isoamyl alcohol (24:1). To the aqueous layer, 0.1 volume of 7.5 M ammonium acetate and 2.5 volume of ethanol were added and incubated for 15 min. at room temperature to precipitate single stranded DNA. The DNA was recovered by centrifugation and suspended in 200 µl TE.

### 3. Mutagenesis of D⁸ and D¹³⁷

Two oligos were designed to mutagenize D⁸ and D¹³⁷ to alanine. The oligos are: 5' GTAGGCCAGGGCTGT**GCCGGC**AAAGAGAAATAGTC 3' (SEQ ID NO:19) (D8A) and 5' GAAGCATATCCTT**GGCGCC**GGTTAT TATGAAAATC 3' (SEQ ID NO:20) (D137A). In the D8A oligo a *Ngo*AIV (bold underlined) and in the oligo D137A a *Kas*I (bold underlined) site was created for easy identification of clones following mutagenesis. 200 pmol of each oligo was kinased according to the Muta-gene protocol (Bio-Rad, Hercules, CA) using 5 units of T4 Kinase (LTI, Rockville, MD). 200 ng of single stranded DNA was annealed with 2 pmol of oligo according to the Muta-gene protocol. The reaction volume was 10 µl. Following the annealing step, complementary DNA synthesis and ligation was carried out using 5 units of wild-type T7 DNA polymerase (U.S. Biochemicals; Cleveland, Ohio) and 0.5 unit T4 ligase (LTI). 1 µl of the reaction was used to transform a MutS *E. coli* (obtained from Dr. Paul Modrich at the Duke University, NC) and selected in agar plates containing ampicillin. A control annealing and synthesis reaction was carried out without addition of any oligo to determine the background. There were 50-60 fold more colonies in the transformation plates with the oligos than without any oligo. Six colonies from each mutagenic oligo directed synthesis were grown and checked for respective restriction site (*Ngo*AIV or *Kas*I*).* For D8A (*Ngo*AIV), 4 out of 6 generated two fragments (3 kb and 4.1 kb). Since pSportTne has an *Ngo*AIV site near the fl intergenic region, the new *Ngo*AIV site within the *Tne* DNA polymerase produced the expected fragments. The plasmid was designated as pSportTneNgoAIV. For D137A (*Kas*I), 5 out of 6 clones produced two expected fragments of 1.1 kb and 6 kb in size. Since pSportTne has another *Kas*I site, the newly created *Kas*I site generated these two expected fragments. The plasmid was designated as pSportTneKasI. Both D8A and D137A mutations have been confirmed by DNA sequencing.

### 4. Reconstruction of the Mutant Polymerase into Expression Vector

During the course of expression of *Tne* DNA polymerase or mutant *Tne* DNA polymerase, a variety of clones were constructed. One such clone was designated as pTTQ Tne SeqS1. This plasmid was constructed as follows: first, similar to above mutagenesis technique glycine 195 was changed to an aspartic acid in pSportTne. A mutation in the corresponding amino acid in *E. coli* DNA polymerase I (polA214, domain F) was found to have lost the 5'-3' exonuclease activity (Gutman and Minton, see above). An *Ssp*I site was created in the mutant polymerase. Second, a 650 bp *Sst*I*-Sph*I fragment containing the G195D mutation was subcloned in pUCTne35FY (see below) to replace the wild type fragment. This plasmid was called pUCTne3022. Finally, the entire mutant *Tne* DNA polymerase was subcloned from pUCTne3022 into pTTQ18 as *Sst*I*-Hind*III fragment to generate pTTQTneSeqS1. To introduce the mutation D8A or D137A in this expression vector, the 650 bp *Sst*I*-Sph*I was replaced with the same *Sst*I-*Sph*I fragment from pSportTneNgoAIV or pSportTneKasI. The plasmids were designated as pTTQTneNgo(D8A) and pTTQTneKas(D137A), respectively.

### 5. Confirmation of the Mutations by DNA Sequencing

DNA sequencing of both mutant polymerases confirmed the presence of the restriction site *Ngo*AIV as well as the mutation D8A; and *Kas*I site as well as the mutation D137A. Also confirmed by DNA sequencing was the presence of the mutation D323A and the *Eco*47III restriction site in the 3'-5'exonuclease region. In addition, confirmed by DNA sequencing was the F730Y mutation and the *Hpa*I restriction site in the O-helix region of the mutant *Tne* DNA polymerase.

### 6. 5'-3'exonuclease Activity of the Mutant Tne DNA Polymerases

The full length mutant DNA polymerase was purified as described above. The 5'-3'exonuclease activity was determined as described in the LTI catalog. Briefly, 1 pmol of labeled (³²P) *Hae*III digested λ DNA (LTI) was used for the assay. The buffer composition is: 25 mM Tris-HCl (pH 8.3), 5 mM MgCl₂, 50 mM NaCl, 0.01% gelatin. The reaction was initiated by the addition of 0, 2, 4, 6 and 10 units of either wild type or mutant *Tne* DNA polymerase in a 50 µl reaction. The reaction mix was incubated for 1 hr at 72°C. A 10 µl aliquot was subjected to PEI-cellulose thin layer chromatography and the label released was quantitated by liquid scintillation. In this assay, both D8A and D137A mutants showed less than 0.01% label release compared to the wild type *Tne* DNA polymerase. The result demonstrates that in both D8A and D137A mutants the 5'-3'exonuclease activity has been considerably diminished. Thus, it has been confirmed for the first time that these two aspartates are involved with the 5'-3' exonuclease activity.

### Example 14: Generation of double mutants, R722K/F730Y, R722Q/F730Y, R722H/F730Y and R722N/F730Y of Tne DNA polymerase

For all mutations PCR method was used. A common 5'-oligo, CAC CAG ACG ***GGTA CC*** GCC ACT GGC AGG TTG (SEQ ID NO:21), was used. This oligo contains a KpnI site (bold italics). The template used for PCR was pTTQTneSeqS1 (Example 13) which already contains the F730Y mutation in the Tne polymerase gene. For R722K/F730Y mutation, the oligo used was TAT AGA GTA ***GTT AAC*** CAT CTT TCC AAC CCG TTT CAT TTC TTC GAA CAC (SEQ ID NO:22). For R722Q/F730Y mutation, the oligo used was TAT AGA GTA ***GTT AAC*** CAT CTT TCC AAC CCG TTG CAT TTC TTC GAA CAC (SEQ ID NO:23). For R722N/F730Y mutation, the oligo used was TAT AGA GTA ***GTT AAC*** CAT CTT TCC AAC CCG GTT CAT TTC TTC GAA CAC (SEQ ID NO:24) and for R722H/F730Y the oligo used was TAT AGA GTA ***GTT AAC*** CAT CTT TCC AAC CCG ATG CAT TTC TTC GAA CAC (SEQ ID NO:25). Each of these oligos contain a HpaI site (bold italics). The underlined codons were the mutated codons for arginine at the position 722 for respective amino acids. The PCR generated a 318 bp product containing a KpnI and a HpaI site. The PCR products were digested with KpnI and HpaI and cloned into pUC-TneFY digested with KpnI and HpaI to replace the original fragment to generate pUC19TneFY-R722K, pUC19TneFY-R722Q, pUC19TneFY-R722H and pUC19TneFY-R722N. Finally, the KpnI-HindIII fragment (~800bp) of pTTQTneKasI(D137A) was replaced by the -800 bp KpnI-HindIII fragment from these plasmids to generate pTne11 (R722K/F730Y), PTne10 (R722Q/F730Y), pTne13 (R722H/F730Y) and pTne9 (R722N/F739Y), respectively. The mutations were confirmed by DNA sequencing.

### Example 15: Generation of Tne DNA Polymerase mutants F730A and F730S (not of the invention)

F730A was constructed using PCR. The forward oligo was AAG ATG ***GTT AAC GCG T***CT ATA ATA TAC GG (SEQ ID NO:26) which contains a HpaI site and a MluI site (bold italics). The reverse oligo was CAA GAG GCA CAG AGA GTT TCA CC (SEQ ID NO:27) which anneals downstream of SpeI present in the Tne polymerase gene. The template used for PCR was pTTQTne KasI (D137A). The 482bp PCR product was digested with HpaI and SpeI and cloned into pUC-TneFY thereby replacing the amino acid tyrosine at position 730 with alanine. This construct was called pUC-Tne FA.

F730S was constructed by site directed mutagenesis. The oligo was GTA TAT TAT AGA GGA ***GTT AAC*** CAT CTT TCC(SEQ ID NO:28) where a HpaI site was created (bold italics). The single stranded DNA used was isolated from pSport-Tne that contains the double mutation D137A and D323A. This construct was designated pTne 47. The Tne polymerase gene was then cloned as an SstI and HindIII fragment into the plasmid pUC19 and the resulting clone was designated pTne101.

### Example 16: Generation of Tne DNA polymerase with a HpaI site in front (not of the invention) of the amino acid phenylalanine at position 730

A construct of Tne polymerase was made using PCR where a HpaI restriction enzyme site was introduced into the gene in front of the amino acid phenylalanine at position 730. The forward oligonucleotide was AAG ATG ***GTT** AAC* TTC TCT ATA ATA TAC GG (SEQ ID NO:29) which contains a HpaI site (bold italics) and the reverse oligo was the same as in example 15 above. The template used for PCR was pTne33 which contains the Tne polymerase gene with D137A and D323A mutations cloned in pUC19. The 482bp PCR product was digested with HpaI and SpeI and was used to replace the corresponding fragment in pTne101 (see example 15). The construct was sequenced to verify that the amino acid at position 730 was indeed phenylalanine and the plasmid was numbered pTne106.

### Example 17: Generation of double mutants R722Y/F730A and R722L/F730A of the Tne DNA polymerase

For both the mutations PCR method was used. The common 5' oligo was the same as in example 14. For R722Y/F730A mutation the oligo used was TAT AGA GTA ***GTT AAC*** CAT CTT TCC AAC CCG GT***A CAT GT**C* TTC GTT CAC (SEQ ID NO:30). For R722L/F730A mutation the oligo used was TAT AGA GTA ***GTT AAC*** CAT CTT TCC AAC CCG CA***A CAT GT*** C TTC GTT CAC (SEQ ID NO:31). Each of these oligos contain a HpaI site (bold italics). The underlined codons were the mutated codons for arginine at the position 722 for respective amino acids. An AflIII site was also created (bold italics next to the underlined codon) in order to confirm the mutation. The PCR generated a 318 bp product containing a KpnI and a HpaI site. The PCR products were digested with KpnI and HpaI and cloned into pUC-TneFA (see example 15). The constructs were named as pUCTneYA and pUCTneLA.

### Example 18: Generation of Tne DNA Polymerase mutants R722Y and R722L

The plasmid pTne 106 (see example 16) was digested with *Hpa*I and *Kpn*I and the 318 bp fragment was replaced with the corresponding fragment from pUCTneYA or pUCTneLA (see example 17) to generate the mutants R722Y or R722L. In these constructs the amino acid at position 730 is the same as wild type Tne (phenylalanine). The constructs were sequenced to confirm the R722Y and the R722L mutations. The *Tne* DNA polymerase gene was then cloned as an SstI/HindIII fragment into the plasmid pSport1.

### Example 19: Generation of Tne DNA Polymerase mutants R722K, R722Q and R722H

The construct pTne 106 (see example 16) was digested with *Hpa*I and *Kpn*I and the 318 bp fragment was replaced with the corresponding fragment from the construct pUC19TneFY-R722K, pUC19TneFY-R722H or pTne10 (see example 14), to generate the mutants R722K, R722H and R722Q. The constructs were sequenced to confirm the mutations. The Tne DNA polymerase gene was then subcloned into the vector pSport1 as an SstI/HindIII fragment.

### Example 20: Purification of the mutant Tne DNA Polymerases

The purification of the mutants of *Tne* DNA polymerase was carried out based on the method described above with minor modifications. Two to three grams of cells expressing cloned mutant *Tne* DNA polymerase were resuspended in 15-20 ml of sonication buffer (50 mM Tris-HCl , pH 8.0, 10% glycerol, 5mM 2-mercaptoethanol, 50 mM NaCl, 1 mM EDTA, and 0.5 mM PMSF and sonicated with a 550 Sonic Dismembrator (Fisher Scientific). The sonicated sample was heated at 82°C for 20 min and then cooled in ice-water for 5 min.

In the sample, 20 mM NaCl and 0.2% PEI were added and centrifuged at 13,000 rpm for 10 min. Ammonium sulfate (305 g/L) was added to the supernatant. The pellet was collected by centrifugation and resuspended in 4 ml of MonoQ column buffer (50 mM Tris-HCl, pH 8.0, 10% glycerol, 5mM 2-mercaptoethanol, 50 mM NaCl, and 1 mM EDTA). The sample was dialyzed against one litter of MonoQ buffer overnight: Following the centrifugation at 13,000 rpm to remove any insoluble materials, the sample was loaded onto a MonoQ column (HR5/5, Pharmacia). The column was washed with MonoQ column buffer to baseline of OD₂₈₀ and then eluted with a linear gradient of 50-300 mM NaCl in 20 ml MonoQ column buffer. The fractions were analyzed by 8% SDS-PAGE and the Tne DNA polymerase activity determined as described earlier. The fractions containing active and pure Tne DNA polymerase were pooled.

### Example 21: Determination of the Fidelity of Mutant Tne DNA Polymerase by Primer Extension Assay

The 34-mer primer 5'-GGGAGACCGGAATTCTCCTTCATT-AATTCCTATA (SEQ ID NO:32) was ³²P labeled at the 5' end with [γ-³²P] ATP (Amersham) and T4 polynucleotide kinase (LTI). The free ATP was removed using a BioRad P6 column (1.0 ml). The labeled primer was annealed to the 84-mer template 5'- ATAAAAGTCACCTGCATCAGCAATAATTGTATATT-GTGGAGACCCTGGAACTATAGGAATTAATGAAGGAGAATTCCGGTCT-CCC (SEQ ID NO:33). Wild-type or mutant *Tne* DNA polymerases (0.125-1.0 unit) were incubated at 72° C for 2 min in a reaction mix containing 25 mM TAPS (pH 9.3), 1.5 mM MgCl₂, 50 mM KCl, 1.0 mM DTT, 40 µM of dCTP, dGTP, dTTP, dATP, and 0.02 pmol of the annealed primer-template. Another set of reactions, in which one of the three dNTPs was omitted, was carried out for all wild-type and mutants of *Tne* DNA polymerase. After addition of sequencing stop buffer and heating at 90°C for 2 min, the mixture was loaded onto a 10% polyacrylamide-7 M urea sequencing gel. Following the electrophoresis, the gel was dried and the reaction products were analyzed with x-ray film autoradiography. Misincorparation of dNTP by wild-type and mutant *Tne* DNA polymerases were quantified by a PhosphorImager(Molecular Dynamics). Results are shown in the table below.

| ***Tne*** | **DNA polymerase** | **Relative Misincorporation, %** |
|---|---|---|
| 1 | D137A, D323A | 100* |
| 2 | D 137A, D323A R722Y | 0.05 |
| 3 | D137A, D323A R722L | 0.05 |
| 4 | D137A, D323A F730Y | 130 |
| 5 | D137A, D323A F730Y R722K | 5 |
| 6 | D137A, D323AF730Y R722H | 4.5 |
| 7 | D137A, D323A F730Y R722N | 6 |
| 8 | D137A, D323A F730Y R722Q | 4.7 |

| | | |
|---|---|---|
| *100% means that 50% of the annealed primer was misextended by the D137A, D323A mutant. | | |

### Example 22: Determination of the Fidelity of Mutant K726R Tne DNA Polymerase (not of the invention)

The mutant K726R was constructed using similar methods as described above in Example 13. An *Eag*I restriction site was introduced into the gene that made a replacement of Lys by Arg at position 726. In 10 µl of reaction mixture, 2.0 pmol of the following oligonucleotide: 5'-pGAAGTTCACCATC**CGGCCGA**CCCGTCGCATTTC-3'(SEQ ID NO:37) was annealed with 200 ng of single-stranded DNA that contained the mutant *Tne* gene containing D137A and D323A mutations. The complementary stranded DNA synthesis and ligation reaction was carried out by addition of 5 units of T7 DNA polymerase (U.S. Biochemicals; Ohio) and 0.5 units of T4 DNA ligase (Life Technologies, Inc.; Rockville, MD). 1 µl of the reaction mixture was used to transform a MutS *E*. *coli* strain, and transformants were selected in agar plates containing ampicillin. Several colonies were grown and plasmid DNA was purified and checked for the *Eag*I restriction site. The resultant mutant *Tne* DNA polymerase was purified as described above in Example 20.

The misincorporation ofdNTPs by this mutant polymerase was determined using the primer extension assay as described in Example 21. The following results were obtained:

| **DNA Polymerase** | **Relative % of Misincorporation** |
|---|---|
| *Tne* D137A, p323A | 100 |
| *Tne* D137A, D323A, K726R | 2.4 |

These results indicate that mutation of the amino acid residue at position 726 of *Tne* DNA polymerase from lysine to arginine resulted in a mutant *Tne* DNA polymerase that was substantially reduced in the ability to misincorporate dNTPs during primer extension.

## Claims

1. A *Tne* DNA polymerase comprising one or more modifications or mutations in the O-helix of the polymerase, the O-helix being RXXXKXXXFXXXYX (SEQ ID NO: 1), wherein X is any amino acid and wherein one mutation or modification is an amino acid substitution at position R (Arg) of the O-helix, whereby R is substituted with Asp (D), Glu (E), His (H), Leu (L), Lys (K) or Tyr (Y) to give increased or enhanced fidelity during nucleic acid synthesis, sequencing or amplification, or reduced or no misincorporation of nucleotides during nucleic acid synthesis compared to corresponding unmutated, unmodified or wild-type enzyme.

2. A *Tne* DNA polymerase as claimed in claim 1, wherein R is substituted with K, H, N or Q.

3. A *Tne* DNA polymerase as claimed in claim 1, wherein R is substituted with K or H.

4. A polymerase as claimed in any of claims 1 to 3, further comprising one or more modifications or mutations to reduce or eliminate an activity selected from the group consisting of:
(a) the 3' →5' exonuclease activity of the polymerase;
(b) the 5'→3' exonuclease activity of the polymerase; and
(c) the discriminatory activity against one or more dideoxynucleotides.

5. A polymerase as claimed in claim 4, which is modified or mutated to reduce or eliminate 3'→5' exonuclease activity.

6. A polymerase as claimed in claim 4, which is modified or mutated to reduce or eliminate discriminatory activity.

7. A polymerase as claimed in claim 4, which is modified or mutated to reduce or eliminate 5'→ 3' exonuclease activity.

8. A polymerase as claimed in any preceding claim further mutated or modified at position K (Lys) of the O-helix; preferably wherein K (Lys) is substituted with an amino acid selected from the group consisting of Ala, Arg, Asn, Asp, Cys, Gln, Glu, Gly, His, Ile, Leu, Met, Phe, Pro, Ser, Thr, Trp, Try and Val

9. A polymerase as claimed in any preceding claim, wherein the misincorporation compared to the unmodified, unmutated or wild-type enzyme is less than 50%.

10. A polymerase as claimed in any preceding claim, wherein the misincorporation compared to the unmodified, unmutated or wild-type enzyme is less than 25%.

11. A polymerase as claimed in any preceding claim, wherein the misincorporation compared to the unmodified, unmutated or wild-type enzyme is less than 10%.

12. A polymerase as claimed in any preceding claim, wherein the misincorporation compared to the unmodified, unmutated or wild-type enzyme is less than 1 %.

13. A method of increasing or enhancing fidelity of nucleic acid synthesis, sequencing or amplification, or reducing or eliminating the misincorporation of nucleotides during nucleic acid synthesis, by a *Tne* DNA polymerase, comprising modifying or mutating the polymerase at the R (Arg) amino acid residue at position 722, wherein R at position 722 is mutated to Y,L,K,H,N or Q.

14. A method as claimed in claim 13, wherein R at position 722 is modified or mutated to K, H, N or Q; preferably K or H.

15. A vector comprising a nucleic acid encoding a polymerase of any of claims 1 to 12.

16. A vector as claimed in claim 15, wherein the nucleic acid is operably linked to a promoter; preferably wherein the promoter is selected from the group consisting of a λ-P_{L} promoter, a tac promoter, a *trp* promoter, and a *trc* promoter.

17. A host cell comprising a vector of claim 15 or claim 16.

18. A method of producing a polymerase, the method comprising:
(a) culturing the host cell of claim 17;
(b) expressing the nucleic acid; and
(c) isolating said polymerase from the host cell.

19. A method as claimed in claim 18, wherein the host cell is *E*. *coli.*

20. A method of synthesizing one or more nucleic acid molecules comprising
(a) mixing a nucleic acid template with polymerase of any of claims 1 to 10 and
(b) incubating the mixture under conditions sufficient to make nucleic acid molecule complementary to all or a portion of the template.

21. A method as claimed in claim 20, wherein the mixture further comprises one or more nucleotides selected from the group consisting of dATP, dCTP, dGTP, dTTP, dITP, 7-deaza-dGTP, dUTP, ddATP, ddCTP, ddGTP, ddITP, ddTTP, [α-S]dATP, [α-S]dTTP, [α-S]dGTP, and [α-S]dCTP; preferably wherein one or more of said nucleotides are detectably labeled.

22. A method of sequencing a DNA molecule, comprising
(a) hybridizing a primer to a first DNA molecule;
(b) contacting the DNA molecule of step (a) with deoxyribonucleoside triphosphates, a DNA polymerase any of claims 1 to 12, and a terminator nucleotide;
(c) incubating the mixture of step (b) under conditions sufficient to synthesize a random population of DNA molecules complementary to the first DNA molecule, wherein the synthesized DNA molecules are shorter in length than the first DNA molecule and wherein the synthesized DNA molecules comprise a terminator nucleotide at their 5' termini; and
(d) separating the synthesized DNA molecules by size so that at least a part of the nucleotide sequence of the first DNA molecule can be determined.

23. A method as claimed in claim 22, wherein the terminator nucleotide is ddTTP, ddATP, ddGTP, ddITP or ddCTP.

24. A method as claimed in claim 22 or claim 23, wherein
(a) one or more of the deoxyribonucleoside triphosphates is detectably labelled; and/or
(b) one or more of the terminator nucleotides is detectably labeled.

25. A method for amplifying a double stranded DNA molecule, comprising
(a) providing a first and second primer, wherein the first primer is complementary to a sequence at or near the 3'-termini of the first strand of the DNA molecule, and the second primer is complementary to a sequence at or near the 3' -termini of the second strand of the DNA molecule;
(b) hybridizing the first primer to the first strand and the second primer to the second strand in the presence of the DNA polymerase of any of claims 1 to 12, under conditions such that a third DNA molecule complementary to the first strand and a fourth DNA molecule complementary to the second strand are synthesized;
(c) denaturing the first and third strand, and the second and fourth strands; and
(d) repeating steps (a) to (c) one or more times.

26. A method as claimed in claim 22, wherein the deoxyribonucleoside triphosphates are selected from the group consisting of dATP, dCTP, dGTP, dTTP, dLTP; 7-deaza-dGTP, dUTP, [α-S]dATP, [α-S]dTTP, [α-S]dGTP, and [α-S]dCTP.

27. A kit for amplifying, synthesizing, or sequencing a DNA molecule comprising: one or more polymerases of any of claims 1 to 12.

28. A kit as claimed in claim 27, further comprising one or more dideoxyribonucleoside triphosphates and/or more deoxyribonucleoside triphosphates.

29. A method of preparing cDNA from mRNA, comprising
(a) mixing one or more mRNA templates with one or more of the polymerases of any of claims 1 to 12; and
(b) incubating the mixture under conditions sufficient to synthesize one or more cDNA molecules complementary to all or a portion of the templates.

30. A method as claimed in claim 27, further comprising incubating the synthesized cDNA under conditions to make double stranded cDNA.

## Patentansprüche

1. Eine The-DNA-Polymerase, die eine oder mehr als eine Modifikation oder Mutation in der O-Helix der Polymerase umfasst, wobei die O-Helix RXXXKXXXFXXXYX (SEQ ID NR: 1) ist, worin X eine beliebige Aminosäure ist und worin eine Mutation oder Modifikation eine Aminosäuresubstitution an der Position R (Arg) der O-Helix ist, wodurch R durch Asp (D), Glu (E), His (H), Leu (L), Lys (K) oder Tyr (Y) substituiert ist, um eine erhöhte oder verbesserte Genauigkeit während einer Synthese, Sequenzierung oder Amplifikation einer Nukleinsäure oder einen verringerten oder keinen Fehleinbau von Nukleotiden während einer Nukleinsäuresynthese im Vergleich zu einem entsprechenden nicht mutierten, nicht modifizierten oder Wildtyp-Enzym zu ergeben.

2. Eine The-DNA-Polymerase wie in Anspruch 1 beansprucht, wobei R durch K, H, N oder Q substituiert ist.

3. Eine Tne-DNA-Polymerase wie in Anspruch 1 beansprucht, wobei R durch K oder H substituiert ist.

4. Eine Polymerase wie in einem der Ansprüche 1 bis 3 beansprucht, die ferner eine oder mehr als eine Modifikation oder Mutation umfasst, um eine Aktivität zu verringern oder zu eliminieren, die aus der Gruppe ausgewählt ist, die aus folgendem besteht:
(a) der 3'->5'-Exonuklease-Aktivität der Polymerase;
(b) der 5'→3'-Exonuklease-Aktivität der Polymerase; und
(c) der diskriminatorischen Aktivität gegenüber einem oder mehr als einem Dideoxynukleotid.

5. Eine Polymerase wie in Anspruch 4 beansprucht, die modifiziert oder mutiert ist, um die 3'→5'-Exonuklease-Aktivität zu verringern oder zu eliminieren.

6. Eine Polymerase wie in Anspruch 4 beansprucht, die modifiziert oder mutiert ist, um die diskriminatorische Aktivität zu verringern oder zu eliminieren.

7. Eine Polymerase wie in Anspruch 4 beansprucht, die modifiziert oder mutiert ist, um die 5'→3'-Exonuklease-Aktivität zu verringern oder zu eliminieren.

8. Eine Polymerase wie in einem vorstehenden Anspruch beansprucht, die ferner an der Position K (Lys) der O-Helix mutiert oder modifiziert ist; vorzugsweise wobei K (Lys) durch eine Aminosäure substituiert ist, die aus der Gruppe ausgewählt ist, die aus Ala, Arg, Asn, Asp, Cys, Gin, Glu, Gly, His, Ile, Leu, Met, Phe, Pro, Ser, Thr, Trp, Try und Val besteht.

9. Eine Polymerase wie in einem vorstehenden Anspruch beansprucht, wobei der Fehleinbau im Vergleich mit dem nicht modifizierten, nicht mutierten oder Wildtyp-Enzym weniger als 50 % beträgt.

10. Eine Polymerase wie in einem vorstehenden Anspruch beansprucht, wobei der Fehleinbau im Vergleich mit dem nicht modifizierten, nicht mutierten oder Wildtyp-Enzym weniger als 25 % beträgt.

11. Eine Polymerase wie in einem vorstehenden Anspruch beansprucht, wobei der Fehleinbau im Vergleich mit dem nicht modifizierten, nicht mutierten oder Wildtyp-Enzym weniger als 10% beträgt.

12. Eine Polymerase wie in einem vorstehenden Anspruch beansprucht, wobei der Fehleinbau im Vergleich mit dem nicht modifizierten, nicht mutierten oder Wildtyp-Enzym weniger als 1 % beträgt.

13. Ein Verfahren zur Steigerung oder Verbesserung der Genauigkeit einer Synthese, Sequenzierung oder Amplifikation einer Nukleinsäure oder zur Verringerung oder Eliminierung des Fehleinbaus von Nukleotiden während einer Nukleinsäuresynthese durch eine Tne-DNA-Polymerase, das das Modifizieren oder Mutieren der Polymerase an der R (Arg) Aminosäuregruppe an der Position 722 umfasst, wobei R an der Position 722 zu Y, L, K, H, N oder Q mutiert wird.

14. Ein Verfahren wie in Anspruch 13 beansprucht, wobei R an der Position 722 zu K, H, N oder Q; vorzugsweise K oder H modifiziert oder mutiert wird.

15. Ein Vektor, der eine Nukleinsäure umfasst, die für eine Polymerase nach einem der Ansprüche 1 bis 12 kodiert.

16. Ein Vektor wie in Anspruch 15 beansprucht, wobei die Nukleinsäure operabel an einen Promotor gebunden ist; vorzugsweise wobei der Promotor aus der Gruppe ausgewählt ist, die aus einem λ-P_{L}-Promotor, einem tac-Promotor, einem trp-Promotor und einem trc-Promotor besteht.

17. Eine Wirtszelle, die einen Vektor nach Anspruch 15 oder Anspruch 16 umfasst.

18. Ein Verfahren zur Herstellung einer Polymerase, wobei das Verfahren folgendes umfasst:
(a) Kultivieren der Wirtszelle nach Anspruch 17;
(b) Exprimieren der Nukleinsäure; und
(c) Isolieren der Polymerase aus der Wirtszelle.

19. Ein Verfahren wie in Anspruch 18 beansprucht, wobei die Wirtszelle E. coli ist.

20. Ein Verfahren zur Synthese von einem oder mehr als einem Nukleinsäuremolekül, das folgendes umfasst:
(a) Vermischen eines Nukleinsäuretemplats mit der Polymerase nach einem der Ansprüche 1 bis 10; und
(b) Inkubieren des Gemischs unter Bedingungen, die ausreichend sind, um ein Nukleinsäuremolekül zu erzeugen, das zu der Gesamtheit oder einem Teil des Templats komplementär ist.

21. Ein Verfahren wie in Anspruch 20 beansprucht, wobei das Gemisch ferner ein oder mehr als ein Nukleotid umfasst, das aus der Gruppe ausgewählt ist, die aus dATP, dCTP, dGTP, dTTP, dITP, 7-deaza-dGTP, dUTP, ddATP, ddCTP, ddGTP, ddITP, ddTTP, [α-S]dATP, [α-S]dTTP, [α-S]dGTP und [α-S]dCTP besteht; vorzugsweise wobei eines oder mehr als eines der Nukleotide nachweisbar markiert ist.

22. Ein Verfahren zur Sequenzierung eines DNA-Moleküls, das folgendes umfasst:
(a) Hybridisieren eines Primers an ein erstes DNA-Molekül;
(b) Kontaktieren des DNA-Moleküls aus dem Schritt (a) mit Deoxyribonukleosidtriphosphaten, einer DNA-Polymerase nach einem der Ansprüche 1 bis 12 und einem Terminator-Nukleotid;
(c) Inkubieren des Gemischs aus dem Schritt (b) unter Bedingungen, die ausreichend sind, um eine zufällige Population von DNA-Molekülen zu synthetisieren, die zu dem ersten DNA-Molekül komplementär sind, wobei die synthetisierten DNA-Moleküle der Länge nach kürzer als das erste DNA-Molekül sind und wobei die synthetisierten DNA-Moleküle ein Terminator-Nukleotid an ihren 5'-Enden umfassen; und
(d) Trennen der synthetisierten DNA-Moleküle der Größe nach, so dass mindestens ein Teil der Nukleotidsequenz des ersten DNA-Moleküls bestimmt werden kann.

23. Ein Verfahren wie in Anspruch 22 beansprucht, wobei das Terminator-Nukleotid ddTTP, ddATP, ddGTP, ddITP oder ddCTP ist.

24. Ein Verfahren wie in Anspruch 22 oder Anspruch 23 beansprucht, wobei
(a) eines oder mehr als eines der Deoxyribonukleosidtriphosphate nachweisbar markiert ist; und/oder
(b) eines oder mehr als eines der Terminator-Nukleotide nachweisbar markiert ist.

25. Ein Verfahren zur Amplifikation eines doppelsträngigen DNA-Moleküls, das folgendes umfasst:
(a) Bereitstellen eines ersten und zweiten Primers, wobei der erste Primer komplementär zu einer Sequenz an den oder nahe der 3'-Enden des ersten Strangs des DNA-Moleküls ist und der zweite Primer komplementär zu einer Sequenz an den oder nahe der 3'-Enden des zweiten Strangs des DNA-Moleküls ist;
(b) Hybridisieren des ersten Primers an den ersten Strang und des zweiten Primers an den zweiten Strang bei Vorhandensein der DNA-Polymerase nach einem der Ansprüche 1 bis 12 unter derartigen Bedingungen, dass ein drittes DNA-Molekül, das zu dem ersten Strang komplementär ist, und ein viertes DNA-Molekül, das zu dem zweiten Strang komplementär ist, synthetisiert wird;
(c) Denaturieren des ersten und dritten Strangs sowie des zweiten und vierten Strangs; und
(d) Wiederholen der Schritte (a) bis (c) einmal oder mehr als einmal.

26. Ein Verfahren wie in Anspruch 22 beansprucht, wobei die Deoxyribonukleosidtriphosphate aus der Gruppe ausgewählt sind, die aus dATP, dCTP, dGTP, dTTP, dITP, 7-deaza-dGTP, dUTP, [α-S]dATP, [α-S]dTTP, [α-S]dGTP und [α-S]dCTP besteht.

27. Ein Kit zur Amplifikation, Synthese oder Sequenzierung eines DNA-Moleküls, das eine oder mehr als eine Polymerase nach einem der Ansprüche 1 bis 12 umfasst.

28. Ein Kit wie in Anspruch 27 beansprucht, das ferner ein oder mehr als ein Dideoxyribonukleosidtriphosphat und/oder mehr als ein Deoxyribonukleosidtriphosphat umfasst.

29. Ein Verfahren zur Herstellung von cDNA aus mRNA, das folgendes umfasst:
(a) Vermischen von einem oder mehr als einem mRNA-Templat mit einem oder mehr als einem der Polymerasen nach einem der Ansprüche 1 bis 12; und
(b) Inkubieren des Gemischs unter Bedingungen, die ausreichend sind, um ein oder mehr als ein cDNA-Molekül zu synthetisieren, das zu der Gesamtheit oder einem Teil der Template komplementär ist.

30. Ein Verfahren wie in Anspruch 27 beansprucht, das ferner das Inkubieren der synthetisierten cDNA unter Bedingungen umfasst, um doppelsträngige cDNA herzustellen.

## Revendications

1. ADN polymérase Tne comprenant une ou plusieurs modifications ou mutations dans l'hélice O de la polymérase, l'hélice O étant RXXXKXXXFXXXYX (SEQ ID NO : 1), où X représente n'importe quel acide aminé et où une mutation ou modification est une substitution d'acide aminé en position R (Arg) de l'hélice O, R étant substitué par Asp (D), Glu (E), His (H), Leu (L), Lys (K) ou Tyr (Y) pour donner une fidélité accrue ou améliorée pendant la synthèse, le séquençage ou l'amplification d'acide nucléique, ou une mauvaise incorporation réduite ou nulle de nucléotides pendant la synthèse d'acide nucléique par comparaison avec une enzyme non mutée, non modifiée ou de type sauvage correspondante.

2. ADN polymérase *Tne* selon la revendication 1, dans laquelle R est substitué par K, H, N ou Q.

3. ADN polymérase Tne selon la revendication 1, dans laquelle R est substitué par K ou H.

4. Polymérase selon l'une quelconque des revendications 1 à 3, comprenant en outre une ou plusieurs modifications ou mutations pour réduire ou éliminer une activité choisie dans le groupe consistant en :
(a) l'activité exonucléase 3'→>5' de la polymérase ;
(b) l'activité exonucléase 5'→3' de la polymérase ; et
(c) l'activité discriminatoire à l'encontre d'un ou plusieurs didésoxynucléotides.

5. Polymérase selon la revendication 4, qui est modifiée ou mutée pour réduire ou éliminer l'activité exonucléase 3'→5'.

6. Polymérase selon la revendication 4, qui est modifiée ou mutée pour réduire ou éliminer l'activité discriminatoire.

7. Polymérase selon la revendication 4, qui est modifiée ou mutée pour réduire ou éliminer l'activité exonucléase 5'→3'.

8. Polymérase selon l'une quelconque des revendications précédentes, qui est également encore mutée ou modifiée en position K(Lys) de l'hélice O ; de préférence où K(Lys) est substitué par un acide aminé choisi dans le groupe consistant en Ala, Arg, Asn, Asp, Cys, Gln, Glu, Gly, His, Ile, Leu, Met, Phe, Pro, Ser, Thr, Trp, Try et Val.

9. Polymérase selon l'une quelconque des revendications précédentes, dans laquelle la mauvaise incorporation par comparaison avec l'enzyme non modifiée, non mutée ou de type sauvage est inférieure à 50 %.

10. Polymérase selon l'une quelconque des revendications précédentes, dans laquelle la mauvaise incorporation comparée à l'enzyme non modifiée, non mutée ou de type sauvage est inférieure à 25 %.

11. Polymérase selon l'une quelconque des revendications précédentes, dans laquelle la mauvaise incorporation par comparaison avec l'enzyme non modifiée, non mutée ou de type sauvage est inférieure à 10 %.

12. Polymérase selon l'une quelconque des revendications précédentes, dans laquelle la mauvaise incorporation comparée à l'enzyme non modifiée, non mutée ou de type de sauvage est inférieure à 1 %.

13. Procédé d'augmentation ou d'amélioration de la fidélité de la synthèse, du séquençage ou de l'amplification d'un acide nucléique, ou de réduction ou d'élimination de la mauvaise incorporation de nucléotides pendant la synthèse de l'acide nucléique, par une ADN polymérase Tne, comprenant la modification ou la mutation de la polymérase au résidu d'acide aminé R(Arg) en position 722, où R en position 722 est muté en Y, L, K, H, N ou Q.

14. Procédé selon la revendication 13, dans lequel R en position 722 est modifié ou muté en K, H, N ou Q ; de préférence K ou H.

15. Vecteur comprenant un acide nucléique codant pour une polymérase selon l'une quelconque des revendications 1 à 12.

16. Vecteur selon la revendication 15, dans lequel l'acide nucléique est lié de façon fonctionnelle à un promoteur, de préférence dans lequel promoteur est choisi dans le groupe constitué par un promoteur λ-P_{L}, un promoteur tac, un promoteur *trp* et un promoteur *trc*.

17. Cellule hôte comprenant un vecteur de la revendication 15 ou de la revendication 16.

18. Procédé de production d'une polymérases, le procédé comprenant :
(a) la culture de la cellule hôte de la revendication 17 ;
(b) l'expression de l'acide nucléique ; et
(c) l'isolement de ladite polymérase à partir de la cellule hôte.

19. Procédé selon la revendication 18, dans lequel la cellule hôte est *E. coli*.

20. Procédé de synthèse d'une ou plusieurs molécules d'acide nucléique comprenant :
(a) le mélange d'une matrice d'acide nucléique avec une polymérase de l'une quelconque des revendications 1 à 10 ; et
(b) l'incubation du mélange dans des conditions suffisantes pour rendre une molécule d'acide nucléique complémentaire de la totalité ou d'une partie de la matrice.

21. Procédé selon la revendication 20, dans lequel le mélange comprend en outre un ou plusieurs nucléotides choisis dans le groupe constitué par dATP, dCTP, dGTP, dTTP, dITP, 7-déaza-dGTP, dNTP, ddATP, ddCTP, ddGTP, ddlTP, ddTTP, [α-S] dATP, [α-S] dTTP, [α-S] dGTP et [α-S]dCTP ; de préférence dans lequel un ou plusieurs desdits nucléotides sont marqués de façon détectable.

22. Procédé de séquençage d'une molécule d'ADN, comprenant :
(a) l'hybridation d'une amorce à une première molécule d'ADN ;
(b) la mise en contact de la molécule d'ADN de l'étape (a) avec des désoxyribonucléoside triphosphate, une ADN polymérase telle que définie à l'une quelconque des revendications 1 à 12, et un nucléotide terminateur ;
(c) l'incubation du mélange de l'étape (b) dans des conditions suffisantes pour synthétiser une population aléatoire de molécules d'ADN complémentaires de la première molécule d'ADN, les molécules d'ADN synthétisées étant de plus courtes longueurs que la première molécule d'ADN, et les molécules d'ADN synthétisées comprenant un nucléotide terminateur à leurs extrémités terminales 5' ; et
(d) la séparation par dimension de molécules d'ADN synthétisés de telle sorte qu'au moins une partie de la séquence nucléotidique de la première molécule d'ADN peut être déterminée.

23. Procédé selon la revendication 22, dans lequel le nucléotide terminateur est ddTTP, ddATP, ddGTP, ddITP ou ddCTP.

24. Procédé selon la revendication 22 ou la revendication 23, dans lequel :
(a) un ou plusieurs des désoxyribonucléosides triphosphate est marqué de façon détectable ; et/ou
(b) un ou plusieurs des nucléotides terminateurs sont marqués de façon détectable.

25. Procédé d'amplification d'une molécule d'ADN double brin, comprenant les opérations comprenant :
(a) se procurer une première et une seconde amorces, la première amorce étant complémentaire d'une séquence située aux extrémités terminales 3' du premier brin de la molécule d'ADN ou près de celle-ci, et la seconde amorce étant complémentaire d'une séquence située aux extrémités terminales 3' du second brin de la molécule d'ADN ou près de celles-ci;
(b) hybrider la première amorce au premier brin et la seconde amorce au second brin en présence de l'ADN polymérase de l'une quelconque des revendications 1 à 12, dans des conditions telles qu'une troisième molécule d'ADN complémentaire du premier brin et une quatrième molécule d'ADN complémentaire du second brin sont synthétisées ;
(c) la dénaturation des premier et troisième brins, et des second et quatrième brins ; et
(d) la répétition des étapes (a) à (c) une ou plusieurs fois.

26. Procédé selon la revendication 22, dans lequel les désoxyribonucléosides triphosphates sont choisis dans le groupe constitué par dATP, dCTP, dGTP, dTTP, dITP, 7-déaza-dGTP, dUTP, [α-S]dATP, [α-S] dTfP, [α-S] dGTP et [α-S]dCTP.

27. Coffret pour amplifier, synthétiser ou séquencer une molécule d'ADN comprenant une ou plusieurs polymérases de l'une quelconque des revendications 1 à 2.

28. Coffret selon la revendication 27, comprenant en outre un ou plusieurs didésoxyribonucléosides triphosphates et/ou davantage de désoxyribonucléosides triphosphates.

29. Procédé de préparation d'un ADNc à partir d'un ARNm comprenant :
(a) le mélange d'une ou plusieurs matrices d'ARNm avec une ou plusieurs des polymérases de l'une quelconque des revendications 1 à 12 ; et
(b) l'incubation d'un mélange dans des conditions suffisantes pour synthétiser une ou plusieurs d'ADNc complémentaire de la totalité ou d'une partie des matrices.

30. Procédé selon la revendication 27, comprenant en outre l'incubation de l'ADNc dans des conditions pour fabriquer un ADN_{C} double brin.


### SEQUENCE LISTING

<110> Life Technologies, Inc.
   9800 Medical Center Drive
   Rockville, Maryland 20850
   United States of America
<120> High Fidelity Polymerases and Uses Thereof
<130> 0942.450PC03
<140>
   <141>
<150> 60/056,263
   <151> 1997-08-29
<150> 60/060,131
   <151> 1997-09-26
<150> 60/085,247
   <151> 1998-05-13
<160> 37
<170> PatentIn Ver. 2.0
<210> 1
   <211> 14
   <212> PRT
   <213> Escherichia coli
<400> 1
   Arg Xaa Xaa Xaa Lys Xaa Xaa Xaa Phe Xaa Xaa Xaa Tyr Xaa
    1               5                   10
<210> 2
   <211> 14
   <212> PRT
   <213> Escherichia coli
<400> 2
   Arg Arg Ser Ala Lys Ala Ile Asn Phe Gly Leu Ile Tyr Gly
    1               5                   10
<210> 3
   <211> 14
   <212> PRT
   <213> Thermus aquaticus
<400> 3
   Arg Arg Ala Ala Lys Thr Ile Asn Phe Gly Val Leu Tyr Gly
    1               5                   10
<210> 4
   <211> 14
   <212> PRT
   <213> Bacteriophage T7
<400> 4
   Arg Asp Asn Ala Lys Thr Phe Ile Tyr Gly Phe Leu Tyr Gly
    1               5                   10
<210> 5
   <211> 14
   <212> PRT
   <213> Thermotoga neapolitana
<400> 5
   Arg Arg Val Gly Lys Met Val Asn Phe Ser Ile Ile Tyr Gly
    1               5                  10
<210> 6
   <211> 14
   <212> PRT
   <213> Bacteriophage T5
<400> 6
   Arg Gln Ala Ala Lys Ala Ile Thr Phe Gly Ile Leu Tyr Gly
    1               5                  10
<210> 7
   <211> 14
   <212> PRT
   <213> Thermotoga maritima
<400> 7
   Arg Arg Ala Gly Lys Met Val Asn Phe Ser Ile Ile Tyr Gly
    1               5                  10
<210> 8
   <211> 11
   <212> PRT
   <213> Thermotoga neapolitana
<400> 8
   Pro Ser Phe Ala Leu Asp Leu Glu Thr Ser Ser
    1               5                  10
<210> 9
   <211> 11
   <212> PRT
   <213> Escherichia coli
<400> 9
   Pro Val Phe Ala Phe Asp Thr Glu Thr Asp Ser
   1 5 10
<210> 10
   <211> 11
   <212> PRT
   <213> Bacteriophage T5
<400> 10
   Gly Pro Val Ala Phe Asp Ser Glu Thr Ser Ala
   1 5 10
<210> 11
   <211> 10
   <212> PRT
   <213> Bacteriophage T7
<400> 11
   Met Ile Val Ser Asp Ile Glu Ala Asn Ala
   1 5 10
<210> 12
   <211> 26
   <212> DNA
   <213> Artificial sequence
<220>
   <223> Description of artificial sequence: synthetic oligonucleotide
<400> 12
   gacgtttcaa gcgctagggc aaaaga 26
<210> 13
   <211> 14
   <212> PRT
   <213> Thermotoga neapolitana
<400> 13
   Arg Arg Val Gly Lys Met Val Asn Phe Ser Ile Ile Tyr Gly
   1 5 10
<210> 14
   <211> 31
   <212> DNA
   <213> Artificial sequence
<220>
   <223> Description of artificial sequence: synthetic oligonucleotide
<400> 14
   gtatattata gagtagttaa ccatctttcc a 31
<210> 15
   <211> 6
   <212> PRT
   <213> Thermotoga neapolitana
<400> 15
   Phe Leu Phe Asp Gly Thr
   1 5
<210> 16
   <211> 6
   <212> PRT
   <213> Thermus aquaticus
<400> 16
   Leu Leu Val Asp Gly His
   1 5
<210> 17
   <211> 10
   <212> PRT
   <213> Thermotoga neapolitana
<400> 17
   Ser Leu Ile Thr Gly Asp Lys Asp Met Leu
   1 5 10
<210> 18
   <211> 10
   <212> PRT
   <213> Thermus aquaticus
<400> 18
   Arg Ile Leu Thr Ala Asp Lys Asp Leu Tyr
   1 5 10
<210> 19
   <211> 35
   <212> DNA
   <213> Artificial sequence
<220>
   <223> Description of artificial sequence: synthetic oligonucleotide
<400> 19
   gtaggccagg gctgtgccgg caaagagaaa tagtc 35
<210> 20
   <211> 35
   <212> DNA
   <213> Artificial sequence
<220>
   <223> Description of artificial sequence: synthetic oligonucleotide
<400> 20
   gaagcatatc cttggcgccg gttattatga aaatc 35
<210> 21
   <211> 27
   <212> DNA
   <213> Artificial sequence
<220>
   <223> Description of artificial sequence: synthetic oligonucleotide
<400> 21
   caccagacgggta ccgccactgg caggttg 27
<210> 22
   <211> 48
   <212> DNA
   <213> Artificial sequence
<220>
   <223> Description of artificial sequence: synthetic oligonucleotide
<400> 22
   tatagagtag ttaaccatct ttccaacccg tttcatttct tcgaacac 48
<210> 23
   <211> 48
   <212> DNA
   <213> Artificial sequence
<220>
   <223> Description of artificial sequence: synthetic oligonucleotide
<400> 23
   tatagagtag ttaaccatct ttccaacccg ttgcatttct tcgaacac 48
<210> 24
   <211> 48
   <212> DNA
   <213> Artificial sequence
<220>
   <223> Description of artificial sequence: synthetic oligonucleotide
<400> 24
   tatagagtag ttaaccatct ttccaacccg gttcatttct tcgaacac 48
<210> 25
   <211> 48
   <212> DNA
   <213> Artificial sequence
<220>
   <223> Description of artificial sequence: synthetic oligonucleotide
<400> 25
   tatagagtag ttaaccatct ttccaacccg atgcatttct tcgaacac 48
<210> 26
   <211> 29
   <212> DNA
   <213> Artificial sequence
<220>
   <223> Description of artificial sequence: synthetic oligonucleotide
<400> 26
   aagatggtta acgcgtctat aatatacgg 29
<210> 27
   <211> 23
   <212> DNA
   <213> Artificial sequence
<220>
   <223> Description of artificial sequence: synthetic oligonucleotide
<400> 27
   caagaggcac agagagtttc acc 23
<210> 28
   <211> 30
   <212> DNA
   <213> Artificial sequence
<220>
   <223> Description of artificial sequence: synthetic oligonucleotide
<400> 28
   gtatattata gaggagttaa ccatctttcc 30
<210> 29
   <211> 29
   <212> DNA
   <213> Artificial sequence
<220>
   <223> Description of artificial sequence: synthetic oligonucleotide
<400> 29
   aagatggtta acttctctat aatatacgg 29
<210> 30
   <211> 48
   <212> DNA
   <213> Artificial sequence
<220>
   <223> Description of artificial sequence: synthetic oligonucleotide
<400> 30
   tatagagtag ttaaccatct ttccaacccg gtacatgtct tcgttcac 48
<210> 31
   <211> 48
   <212> DNA
   <213> Artificial sequence
<220>
   <223> Description of artificial sequence: synthetic oligonucleotide
<400> 31
   tatagagtag ttaaccatct ttccaacccg caacatgtct tcgttcac 48
<210> 32
   <211> 34
   <212> DNA
   <213> Artificial sequence
<220>
   <223> Description of artificial sequence: synthetic oligonucleotide
<400> 32
   gggagaccgg aattctcctt cattaattcc tata 34
<210> 33
   <211> 85
   <212> DNA
   <213> Artificial sequence
<220>
   <223> Description of artificial sequence: synthetic oligonucleotide
<400> 33
   ataaaagtca cctgcatcag caataattgt atattgtgga gaccctggaa ctataggaat 60
   taatgaagga gaattccggt ctccc 85
<210> 34
   <211> 27
   <212> DNA
   <213> Artificial sequence
<220>
   <223> Description of artificial sequence: synthetic oligonucleotide
<400> 34
   cttggccgcc cgatgcatca gggggtc 27
<210> 35
   <211> 30
   <212> DNA
   <213> Artificial sequence
<220>
   <223> Description of artificial sequence: synthetic oligonucleotide
<400> 35
   cttggccgcc cgcttcatga gggggtccac 30
<210> 36
   <211> 27
   <212> DNA
   <213> Artificial sequence
<220>
   <223> Description of artificial sequence: synthetic oligonucleotide
<400> 36
   cttggccgcc ctgtacatca gggggtc 27
<210> 37
   <211> 33
   <212> DNA
   <213> Artificial sequence
<220>
   <223> Description of artificial sequence: synthetic oligonucleotide
<400> 37
   gaagttcacc atccggccga cccgtcgcat ttc 33